# EUROPEAN PATENT SPECIFICATION

(11) **EP 3 359 183 B1**
(45) Date of publication and mention of the grant of the patent: **17.06.2020**
(21) Application number: 16779055.9
(22) Date of filing: 06.10.2016
(51) Int. Cl.: A61K 39/00, A61K 39/12, C12N 15/85, C07K 14/47

(54) **POLYEPITOPE CONSTRUCTS FOR USE IN IMMUNOTHERAPY**
POLYEPITOPKONSTRUKTE ZUR VERWENDUNG IN DER IMMUNTHERAPIE
POLYÉPITOPES RECOMBINANTS DESTINÉS À ÊTRE UTILISÉS EN IMMUNOTHÉRAPIE

(30) Priority: 06.10.2015 EP 15306567
(43) Date of publication of application: 15.08.2018
(73) Proprietor: Invectys, 75015 Paris (FR)
(72) Inventor: LANGLADE DEMOYEN, Pierre, 92200 Neuilly-sur-Seine (FR); HUET, Thierry, 94130 Nogent sur Marne (FR); WAIN-HOBSON, Simon, 78180 Montigny-le-Bretonneux (FR); KOSTRZAK, Anna, 75015 Paris (FR)
(74) Representative: Cabinet Becker et Associés
(86) International application number: PCT/EP2016/073893
(87) International publication number: WO 2017/060360

(56) References cited:
- WO-A1-2013/135553
- WO-A1-2015/063117
- WO-A2-2007/014740
- WO-A2-2014/144885
- SMAHEL M ET AL: "The effect of helper epitopes and cellular localization of an antigen on the outcome of gene gun DNA immunization", GENE THERAPY, vol. 21, no. 2, 1 February 2014 (2014-02-01), pages 225-232, XP055257681, GB ISSN: 0969-7128, DOI: 10.1038/gt.2013.81

## Description

The present invention pertains to the field of immunotherapy and vaccination.

### Background of the invention

Anti-cancer efficient T cell-based immunotherapy must involve both CD8 and CD4 tumor-reactive T-cell responses which recognize tumor specific antigens. CD8 cytotoxic T lymphocytes (CTLs) are considered to be the main actors of the cell-mediated immune response as they exhibit cytotoxic activity against tumor cells expressing tumor associated antigens (TAAs). However, this immune response is efficient only if CD4 T helper 1 (Th1) cells are concomitantly stimulated. These CD4 cells are critical for the induction and maintenance of CD8 T-cells against tumors by providing help through multiple interactions and orchestrating the overall antitumor response (Shedlock and Shen, 2003). Consequently, increasing attention has focused on identifying MHC class I and II epitopes from multiple TAAs (Cheever MA, et al., 2009). During the past few years, human telomerase reverse transcriptase (hTERT) has emerged as the first common tumor antigen and is actively investigated as a universal target for cancer immunotherapy. Human telomerase reverse transcriptase (hTERT) is the catalytic subunit of the telomerase enzyme that synthesizes telomeric DNA at the chromosome ends. hTERT is overexpressed in more than 85% of human tumors from diverse cancer phenotypes, with little or no expression in normal somatic cells (Shay and Bacchetti, 1997).

Clinical trials with hTERT peptide vaccine formulations mainly targeting CD8 epitopes have illustrated that a hTERT-specific immune response can be safely induced in cancer patients and had a noticeable impact on short-term clinical outcomes (Bernhardt et al., 2006; Bolonaki et al, 2007). However, this therapeutic approach failed to induce long-term clinical benefits due to the absence of a sustained immune response. The memory response obtained with hTERT peptide vaccines aimed at targeting CD8 epitopes and especially with short peptides was very low and not persistent. These suboptimal results can be explained in part by a low level or the absence of CD4 T-cell help.

There is still a need for effective therapeutic vaccines against infectious agents or tumors, especially those expressing weakly immunogenic antigens.

### Summary of the invention

The inventors have now developed a DNA vaccine strategy which does not show the drawbacks of the peptide (even long peptide) vaccination, restricted to certain epitopes of telomerase reverse transcriptase (TERT). Particularly, DNA vaccination avoids expensive and complicated procedures for protein production and purification. The constructions of the invention induce both CTL and CD4 helper T-cells independently of the HLA-restriction of the patient, while being safe and inducing a better quantitative and qualitative immune response.

A subject of the invention is a DNA expression vector or a mixture of DNA expression vectors which encodes at least two CD4 epitopes of telomerase reverse transcriptase (TERT) and at least one tumor, viral, bacterial, or parasitic CD8 epitope.

According to a first embodiment, it is provided a mixture of DNA expression vectors which comprises i) at least a DNA expression vector which encodes said at least two CD4 epitopes of TERT and ii) at least a DNA expression vector which encodes said at least one tumor, viral, bacterial, or parasitic CD8 epitope.

According to another embodiment, it is provided a DNA expression vector, which encodes said at least two CD4 epitopes of TERT and said at least one tumor, viral, bacterial, or parasitic CD8 epitope.

Another subject of the invention is a kit comprising
a. a first container containing at least a DNA expression vector which encodes said at least two CD4 epitopes of TERT and
b. a second container containing at least a DNA expression vector which encodes said tumor, viral, bacterial, or parasitic CD8 epitope.

Said DNA expression vector, mixture of DNA expression vectors, or kit, are useful in treating a tumor or an infection in a patient.

### FIGURE LEGENDS

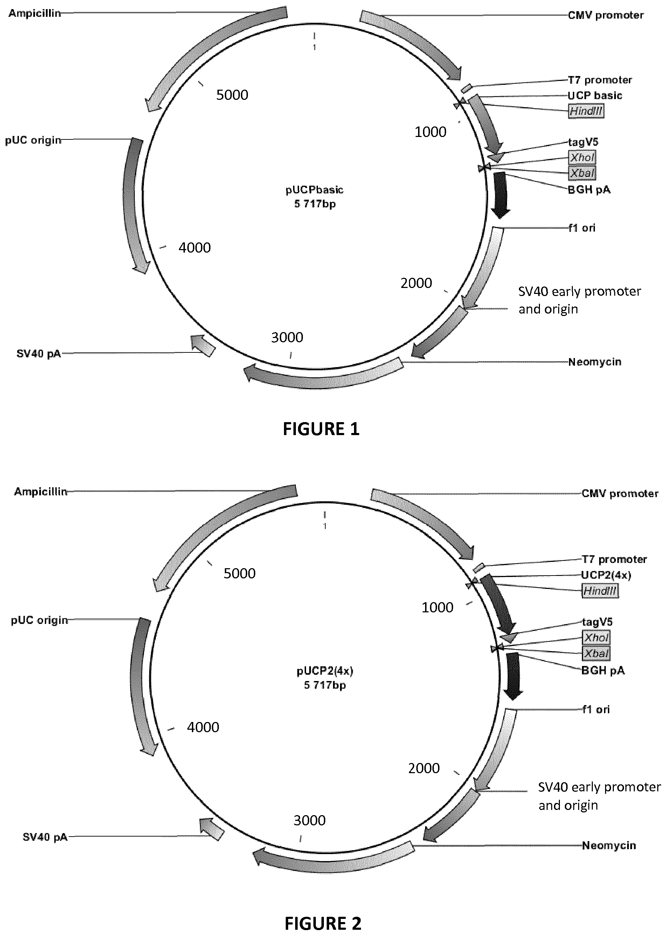

**Figure 1****: pUCPbasic plasmid map**
Table 1 below shows the detailed legend.

| **Gene** | **Location (bases)** |
|---|---|
| CMV promoter | 232-819 |
| T7 promoter | 863-882 |
| UCP basic (4 human TERT class II epitopes) | 923-1225 |
| V5 tag | 1226-1267 |
| BGH pA (BGH polyadenylation sequence) | 1317-1541 |
| f1 ori (f1 origin) | 1587-2015 |
| SV40 early promoter and origin | 2020-2363 |
| Neomycin gene | 2425-3219 |
| SV40 pA (SV40 early polyadenylation signal) | 3393-3523 |
| pUC origin (complementary strand) | 3906-4576 |
| Ampicillin gene (complementary strand) | 4721-5581 |

**Figure 2****: pUCP2(4x) plasmid map**
Table 2 below shows the detailed legend.

| **Gene** | **Location (bases)** |
|---|---|
| CMV promoter | 232-819 |
| T7 promoter | 863-882 |
| UCP2(4x) (human TERT HLA-DRB1 class II epitope repeated four times) | 923-1225 |
| V5 tag | 1226-1267 |
| BGH pA (BGH polyadenylation sequence) | 1317-1541 |
| f1 ori (f1 origin) | 1587-2015 |
| SV40 early promoter and origin | 2020-2363 |
| Neomycin gene | 2425-3219 |
| SV40 pA (SV40 early polyadenylation signal) | 3393-3523 |
| pUC origin (complementary strand) | 3906-4576 |
| Ampicillin gene (complementary strand) | 4721-5581 |

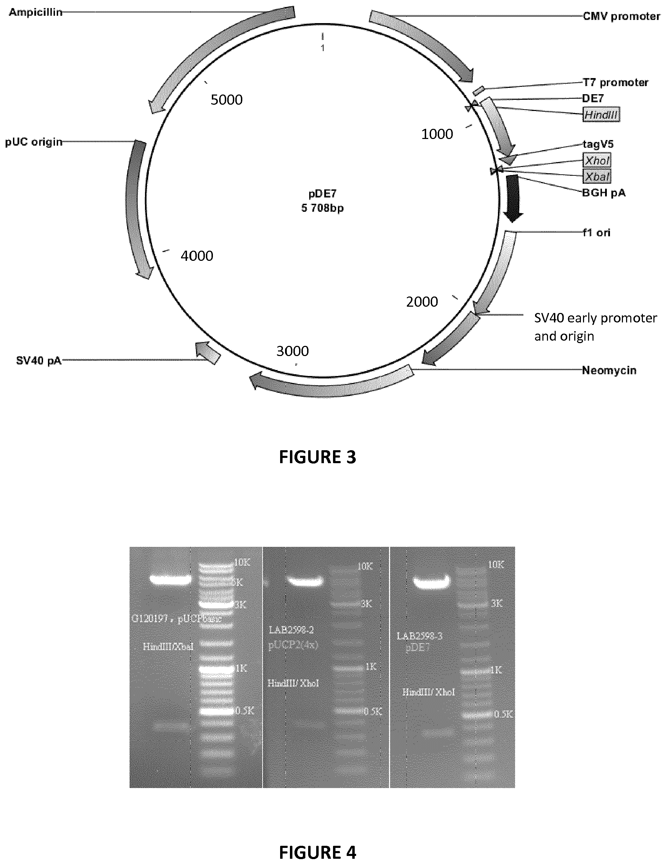

**Figure 3****: pDE7 plasmid map**
Table 3 below shows the detailed legend.

| **Gene** | **Location (bases)** |
|---|---|
| CMV promoter | 232-819 |
| T7 promoter | 863-882 |
| DE7 (HPV 16 non-oncogenic E7 antigen) | 923-1216 |
| V5 tag | 1217-1258 |
| BGH pA (BGH polyadenylation sequence) | 1308-1532 |
| f1 ori (f1 origin) | 1578-2006 |
| SV40 early promoter and origin | 2011-2354 |
| Neomycin gene | 2416-3210 |
| SV40 pA (SV40 early polyadenylation signal) | 3384-3514 |
| pUC origin (complementary strand) | 3897-4567 |
| Ampicillin gene (complementary strand) | 4712-5572 |

**Figure 4****: Validation of the DNA constructs by restriction mapping**
Expression vectors were verified by restriction mapping. Their patterns correspond to expected restriction map.
Lane M: 1 kb Ladder (Eurogentec)
Lane 1: pUCPbasic (369, 5348 bp)
Lane 2: pUCP2(4x) (363, 5354 bp)
Lane 3: pDE7 (354, 5354 bp)

**Figure 5****: Expression of UCPbasic, UCP2(4x) and DE7 polypeptides/proteins *in vitro* in HEK293T cell line assessed by western blotting**
Protein expression was monitored 48 h post-transfection in HEK293T cells.
**(a)** Direct western blotting assay: pUCPbasic, pUCP2(4x), pDE7 and pcDNA3.1 polypeptides/proteins detected after 20 seconds of acquisition; β-actin was used as a loading control.
**(b)** Western blotting assay after immunoprecipitation of E7 protein: whole cell extracts prepared from cells transfected with pDE7 and pcDNA3.1 were immunoprecipitated with anti-V5 agarose beads. The E7 protein was detected after 4 minutes of acquisition.

Molecular weight (MW) markers are indicated (kDa).

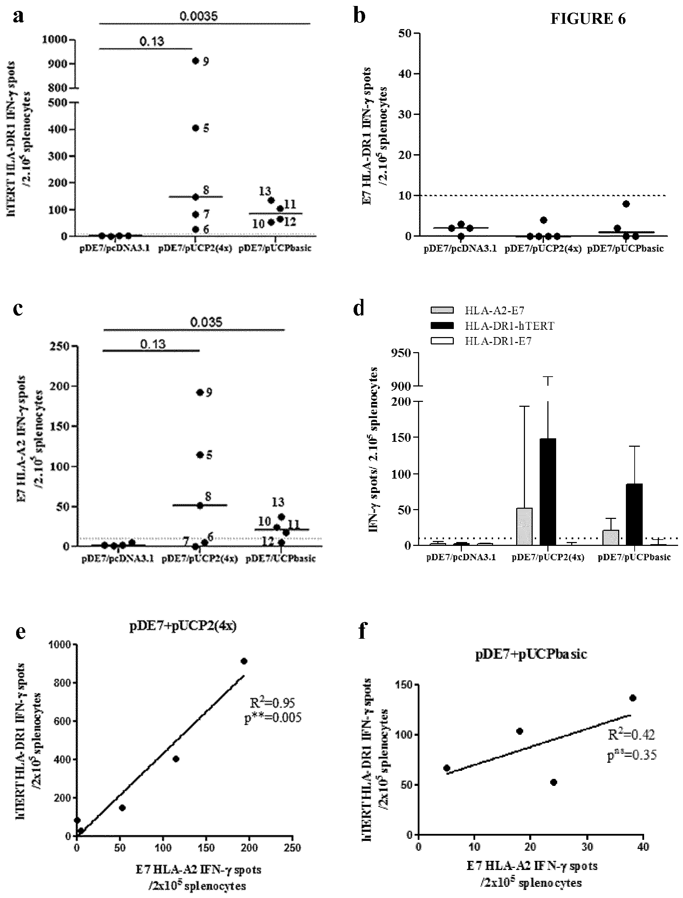

**Figure 6****: hTERT helper epitopes increase the CD8 T-cell response against HPV16 E7 antigen**
Mice were co-immunized with DNA encoding the non-oncogenic E7 antigen and with empty control plasmid (pcDNA3.1), pUCP2(4x) or pUCPbasic at day 0 and day 21. Ten days after the second immunization (D31), spleens were collected and analyzed by ELISpot IFN-γ assay.
Median of the frequency of hTERT and E7 HLA-DR1 (**a, b,** respectively) and E7 HLA-A2 (**c**) restricted IFN-γ+ T-cells in spleen. 5-13: mouse number. (**d**) Aggregated HLA-DR1 and HLA-A2 ELISpot IFN-γ results. Bars show medians with range of IFN- γ spots.
The black horizontal dashed lines indicate the ELISpot positivity threshold. A p value < 0.05 was considered significant (unpaired t test).
Correlation between hTERT HLA-DR1 T-cell and E7 HLA-A2 T-cell responses for pUCP2(4x) + pDE7 (**e**) and pUCPbasic + pDE7 constructs (**f**) determined by the Pearson's correlation coefficient test. A p value < 0.05 was considered significant.
This experimental study was repeated in the same conditions and similar results were obtained.

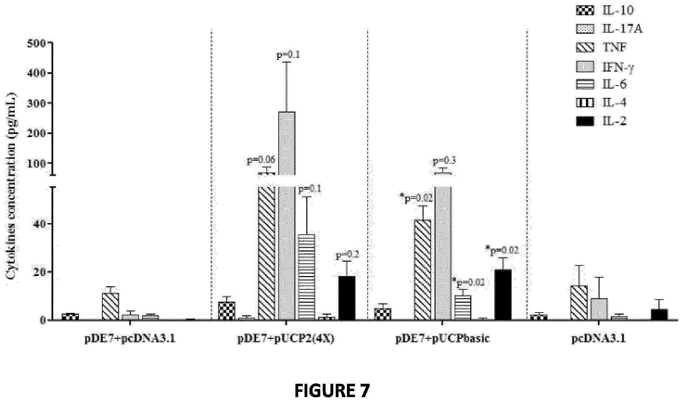

**Figure 7****:** Detection of Thl, Th2 and Th17 cytokine productions by cytokine binding assay (CBA) in splenocyte culture supernatants after 24 hours of culture in the presence of the four hTERT HLA-DR1 peptides of pool 1. Cytokine concentrations in pg/mL are represented as mean ±SD. Statistical analysis: Mann-Whitney non-parametric test against pDE7 + pcDNA3.1 control group. A p value < 0.05 was considered significant (*).

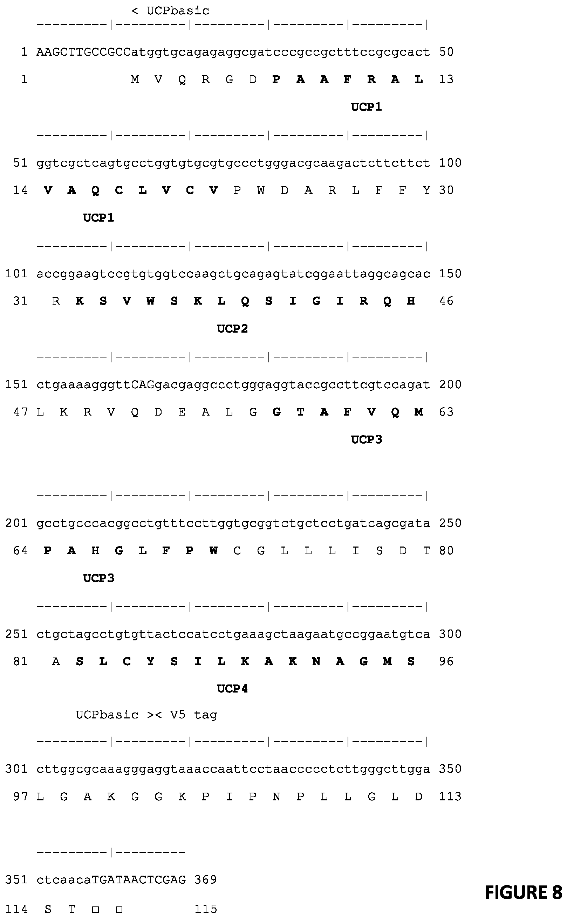

**Figure 8****:** shows the pUCPbasic insert sequence.
Transgene encoding the UCPbasic polypeptide. Four CD4+ human telomerase reverse transcriptase (hTERT; Accession number NM_198253) epitopes, namely UCP1, UCP2, UCP3 and UCP4, with 5 AA natural flanking sequences are linked together. The 14 amino acids at the C-terminal sequence code for the V5 epitope tag. First line is the nucleotide sequence; Second line is the corresponding amino acid sequence. Annotations are given either above or below sequences. □: Stop codon.
Sequence was translated by SHOWORF translation program (EMBOSS Explorer).

| | |
|---|---|
| 1-6 | *Hind*III restriction site for subcloning |
| 13-315 | UCPbasic - UCP1, UCP2, UCP3 and UCP4 **(in bold)** |
| 316-357 | V5 epitope tag |
| 358-363 | two stop codons |
| 364-369 | *Xho*I restriction site for subcloning |

The nucleotide sequence is shown as SEQ ID NO:21, the corresponding amino acid sequence is shown as SEQ ID NO:22.

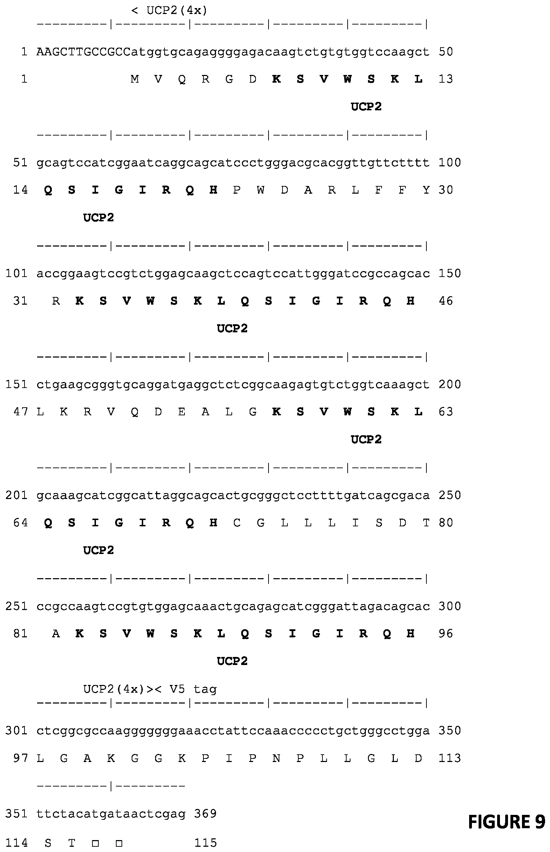

**Figure 9** shows pUCP2(4x) insert sequence.
pUCP2(4x) transgene sequence includes four times repeated UCP2 dominant epitope KSVWSKLQSIGIRQH (SEQ ID NO:2, TERT 578-592, Acc. Nr NM_198253) with 5 AA flanking sequences on each side. The 14 amino acids at the C-terminal sequence code for the V5 epitope tag. First line is the nucleotide sequence; Second line is the corresponding amino acid sequence. Annotations are given either above or below sequences. □: Stop codon.
Sequence was translated by SHOWORF translation program (EMBOSS Explorer).

| | |
|---|---|
| 1-6 | *Hind*III restriction site for subcloning |
| 13-315 | UCP2(4x) - UCP2 unit **(in bold)** |
| 316-357 | V5 epitope tag |
| 358-363 | two stop codons |
| 364-369 | *Xho*I restriction site for subcloning |

The nucleotide sequence is shown as SEQ ID NO:23, the corresponding amino acid sequence is shown as SEQ ID NO:24.

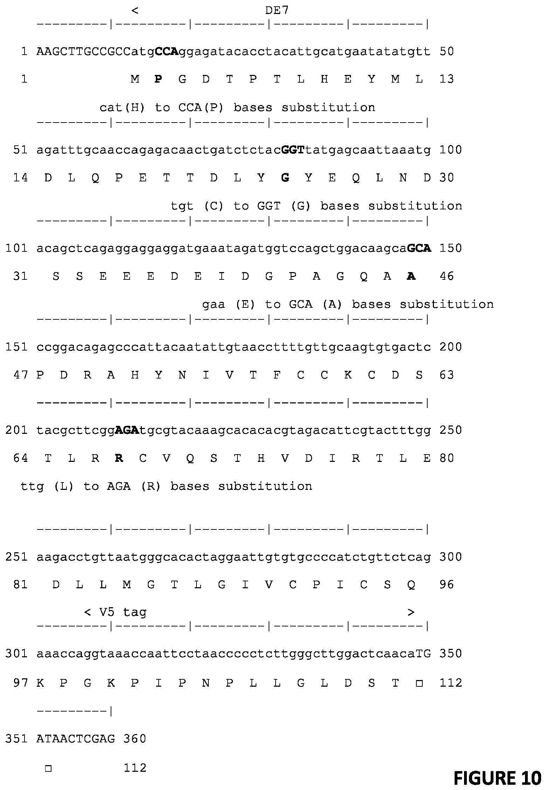

**Figure 10** shows pDE7 insert sequence.
Transgene encoding the DE7 protein. Four mutations were introduced in the wild type E7 gene of human papillomavirus type 16 (Accession number EU869317). The 14 amino acids at the C-terminal sequence code for the V5 epitope tag. First line is the nucleotide sequence; Second line is the corresponding amino acid sequence. Annotations are given either above or below sequences. □: Stop codon. The changed/mutated bases are highlighted in bold.
Sequence was translated by SHOWORF translation program (EMBOSS Explorer).

| | |
|---|---|
| 1-6 | *Hind*III restriction site for subcloning |
| 13-306 | DE7 |
| 307-348 | V5 epitope tag |
| 349-354 | two stop codons |
| 355-360 | *Xho*I restriction site for subcloning |

The nucleotide sequence is shown as SEQ ID NO:25, the corresponding amino acid sequence is shown as SEQ ID NO:26.

### Detailed description of the invention

The inventors propose to use DNA constructs encoding multiple TERT CD4 promiscuous T helper epitopes as universal boosters to induce a sustained immune response against poorly immunogenic antigens.

The inventors designed two synthetic DNA hTERT-derived CD4 polyepitope constructs, pUCPbasic and pUCP2(4x), encoding either four distinct CD4 epitopes (UCP1, UCP2, UCP3 and UCP4, respectively SEQ ID NO:1 to 4) and the UCP2 immunodominant promiscuous epitope repeated four times, respectively, in order to investigate whether it can provide help for improved E7 CD8 T-cell responses. Intradermal (ID) administration followed by electroporation of these two DNA constructs in HLA-A2/DR1 transgenic mice resulted in a strong hTERT-specific CD4 T-cell response that was preferentially Th1 polarized. When coinjected with a plasmid DNA encoding the poorly immunogenic E7 antigen, the magnitude of the E7-specific CD8 T cell response was dramatically increased.

Based on these results, the inventors concluded that (i) CD4 T helper epitopes encoded by hTERT-derived synthetic DNA constructs are efficiently processed and presented in a HLA-DR1 context in HLA-A2/DR1 transgenic mouse model and that (ii) this hTERT CD4 Th1 polarized response can greatly enhance CTL responses against diverse poorly immunogenic cancer/oncoviral antigens or anti-cancer class I polyepitopes.

### Definitions

The telomerase complex consists of an RNA template and protein components including a reverse transcriptase, designated **"Telomerase Reverse Transcriptase" (TERT),** which is the major determinant of telomerase activity. Wild-type human telomerase (or hTERT) is known (GeneBank Accession number NM_198253).

Two amino acid sequences are **"homologous", "substantially homologous" or "substantially similar"** when one or more amino acid residue are replaced by a biologically similar residue or when greater than 80% of the amino acids are identical, or greater than about 90%, preferably greater than about 95%, are similar (functionally identical). Preferably, the similar or homologous sequences are identified by alignment using, for example, the GCG (Genetics Computer Group, Program Manual for the GCG Package, Version 7, Madison, Wisconsin) pileup program, or any of the programs known in the art (BLAST, FASTA, etc.). By "substituted" or "modified" the present invention includes those amino acids that have been altered or modified from naturally occurring amino acids.

The term **"conservative substitution"** as used herein denotes the replacement of an amino acid residue by another, without altering the overall conformation and function of the peptide, including, but not limited to, replacement of an amino acid with one having similar properties (such as, for example, polarity, hydrogen bonding potential, acidic, basic, shape, hydrophobic, aromatic, and the like). Amino acids with similar properties are well known in the art. For example, arginine, histidine and lysine are hydrophilic-basic amino acids and may be interchangeable. Similarly, isoleucine, a hydrophobic amino acid, may be replaced with leucine, methionine or valine. Neutral hydrophilic amino acids, which can be substituted for one another, include asparagine, glutamine, serine and threonine.

The term **"isolated polynucleotide"** is defined as a polynucleotide removed from the environment in which it naturally occurs. For example, a naturally-occurring DNA molecule present in the genome of a living bacteria or as part of a gene bank is not isolated, but the same molecule separated from the remaining part of the bacterial genome, as a result of, e.g., a cloning event (amplification), is isolated. Typically, an isolated DNA molecule is free from DNA regions (e. g., coding regions) with which it is immediately contiguous at the 5' or 3' end, in the naturally occurring genome. Such isolated polynucleotides may be part of a vector or a composition and still be defined as isolated in that such a vector or composition is not part of the natural environment of such polynucleotide.

The term **"immunogenic"** means that the composition or construct to which it refers is capable of inducing an immune response upon administration. **"Immune response"** in a subject refers to the development of an innate and adaptative immune response, including a humoral immune response, a cellular immune response, or a humoral and a cellular immune response to an antigen. A "humoral immune response" refers to one that is mediated by antibodies. A "cellular immune response" is one mediated by T-lymphocytes. It includes the production of cytokines, chemokines and similar molecules produced by activated T-cells, white blood cells, or both. Immune responses can be determined using standard immunoassays and neutralization assays for detection of the humoral immune response, which are known in the art.

In the context of the invention, the immune response preferably encompasses stimulation or proliferation of cytotoxic CD8 T-cells and/or CD4 T-cells and can be determined using immunoassays such as the ELIspot assay, the *in vivo* cytotoxicity assay or the cytokine secretion binding assay.

As used herein, the term **"treatment"** or **"therapy" or "immunotherapy"** refers to any of the alleviation, amelioration and/or elimination, reduction and/or stabilization (e.g., failure to progress to more advanced stages) of a symptom, as well as delay in progression of the disease, or of a symptom thereof. The term thus includes achievement of an efficient anti tumoral immune response observed in cancer patients.

As used herein, the term **"prevention"** or **"preventing"** refers to the alleviation, amelioration and/or elimination, reduction and/or stabilization (e.g., failure to progress to more advanced stages) of a prodrome, *i.e*. any alteration or early symptom (or set of symptoms) that might indicate the start of a disease before specific symptoms occur.

A cell that **"overexpresses telomerase"** refers to a cell in a subject, which either expresses telomerase, e.g. upon mutation or infection, especially infection by an oncovirus, whereas it does usually not, under normal conditions, or to a cell in a subject which expresses a higher level of telomerase (e.g. upon mutation or infection), when compared to normal conditions. Preferably the cell that overexpresses telomerase shows an increase of expression of at least 5%, at least 10%, at least 20%, 30%, 40%, 50%, 60%, 70%, 80%, or more.

The **"patient"** or **"subject"** is typically a mammal subject, preferably a human subject, of any age, sex, or severity of the condition. Non-human mammals are encompassed, including cats, dogs, horses, etc.

### The nucleic acid constructs

It is herein provided nucleic acid constructs that are designed to allow vaccination in patients. In a first embodiment, a mixture of DNA expression vectors encodes at least two, preferably at least three, CD4 epitopes of telomerase reverse transcriptase (TERT) and at least one tumor, viral, bacterial, or parasitic CD8 epitope. Accordingly, the mixture may comprise at least one DNA expression vector which encodes at least two TERT CD4 epitopes (also designated "polyepitope construct"), along with at least one DNA expression vector which encodes at least one tumor, viral, bacterial, or parasitic CD8 epitope.

In another embodiment, a single DNA expression vector encodes at least two CD4 epitopes of TERT and at least one tumor, viral, bacterial, or parasitic CD8 epitope.

The DNA constructs of the invention, which are preferably double stranded DNA, are in isolated form. The nucleic acid constructs are not a naturally-occurring genomic nucleic acid, in particular it do not comprise introns.

### The CD4 epitopes

The DNA expression vector or mixture of DNA expression vectors encodes at least 2, preferably at least 3, still preferably at least 4 CD4 epitopes of TERT.

TERT is preferably human TERT, or TERT from another species, such as cat TERT, or dog TERT, when the subject to treat is a cat or a dog.

However none of the polyepitope constructs of the invention coincides with the coding sequence of the full length TERT, nor with the "shuffle" constructs described in international patent application WO2015/063117. The CD4 epitopes of TERT and the constructs are devoid of telomerase catalytic activity. The constructs of the invention encode less than 70% of the CD4 epitopes of TERT, still preferably less than 60%, less than 50%, less than 40%.

In a particular embodiment the polyepitope construct encodes a polypeptide of less than 160, preferably less than 120 amino acids, the sequence of which comprises at least two, preferably three, still preferably at least four different CD4 TERT epitope, wherein said epitopes are optionally separated by an amino acid spacer.

The DNA expression vector or mixture of DNA expression vectors encodes between 2 and 30 CD4 epitopes of TERT, preferably between 2 and 20, 3 and 18, 3 and 15, 3 and 12, 4 and 10, or still preferably between 4 and 8, CD4 epitopes of TERT.

The term "epitope of TERT" refers to any amino acid fragment of TERT that is an antigenic determinant, i.e. it is recognized by cells of the immune system and is immunogenic, i.e. it can elicit an immune response.

The term "CD4 epitope of TERT" refers to a TERT fragment that is capable of binding to HLA class II molecules and being presented to CD4 T cells. The most useful CD4 epitopes of TERT are also referred to as "UCPs" or "Universal cancer peptides", which means they are expressed in the majority of tumors. The UCPs encoded by the constructs of the invention are able to bind to a broad range of HLA class II alleles, more particularly to HLA-DR allele but also to HLA-DQ and HLA-DP alleles. The peptides are also referred as "HLA class II peptides". Preferably, it can be recognized, specifically by anti-TERT T-cells. Several immunogenic epitope sequences of TERT have been described. See e.g., international patent application WO2013/135553.

The CD4 epitope sequences encoded by the construct of the invention are peptide sequences of 15 to 20 amino acids deriving from TERT. Preferably, the peptides are peptides of 15 to 17 amino acids deriving from TERT.

The peptides as defined herein are then capable of being presented as a complex with a plurality of HLA class II molecule on the surface of tumor cells or antigen presenting cells, thereby being useful in a majority of patients.

The peptides are capable of generating a CD4 Th cell response, preferably a Th1 cell response, directed against the telomerase protein, and have a helper effect on the cytotoxic activity of CD8 T cells.

Four CD4 epitopes are of particular interest: UCP1, UCP2, UCP3 and UCP4. The amino acid sequences are presented in Table below.

**Table 4: UCPs sequences**

| **Peptides** | **Sequences** |
|---|---|
| UCP1 | PAAFRALVAQCLVCV (SEQ ID NO: 1) |
| UCP2 | KSVWSKLQSIGIRQH (SEQ ID NO: 2) |
| UCP3 | GTAFVQMPAHGLFPW (SEQ ID NO: 3) |
| UCP4 | SLCYSILKAKNAGMS (SEQ ID NO: 4) |

Other CD4 epitopes include substantially homologous peptides deriving from SEQ ID NO: 1, 2, 3 or 4 by one, or more substitutions. Preferably the substitutions are conservative and/or improve the peptide immunogenicity.

Advantageously, at least one of said CD4 epitopes of TERT is selected from the group consisting of UCP1, UCP2, UCP3 and UCP4.

In a particular embodiment, at least two CD4 epitopes encoded by the DNA expression vector or the mixture of DNA expression vectors are distinct from each other. For instance the vector or mixture of vectors may encode a polyepitope sequence comprising UCP1, UCP2, UCP3 and UCP4.

In another particular embodiment at least two CD4 epitopes of TERT encoded by the DNA expression vector or the mixture of DNA expression vectors are identical, and repeated.

For instance, the DNA expression vector or a mixture of DNA expression vectors may encode a repetition at least two, preferably at least three, preferably at least four, UCP2 epitopes.

The polynucleotide units encoding the multiple epitopes can be arranged in any order, consecutively, i.e., the 3' end of the first polynucleotide unit is directly linked to the 5' end of the second polynucleotide unit (and so on), resulting in a polynucleotide encoding a peptide sequence exclusively composed of consecutive epitopes. The multiple epitopes can alternatively be separated by a one-amino acid spacer or a peptide spacer, i.e., meaning that the different polynucleotide units are separated by one or several codon(s) encoding respectively one or several amino acid(s). Typically, the CD4 TERT epitopes can be separated by about four to six Gly amino acids.

The order in which the epitopes are arranged can be determined by the man skilled in the art, according to the following criteria: some orders may facilitate either the transcription and/or the translation of the polynucleotide, may facilitate the transport of the resulting expressed polyepitope in the endoplasmic reticulum (ER), especially if the tridimensional conformation impacts the properties, and may facilitate the processing of the polyepitope in several epitopes or analogues and avoid the processing of overlapping epitopes.

The Experimental section illustrates the invention using UCP1, UCP2, UCP3 and UCP4 (pUCPbasic) and UCP2 repeated 4 times [pUCP2(4x)] polyepitopes as boosters. However several other hTERT HLA-DR (class II) epitopes, which were identified along the hTERT protein sequence by *in silico* prediction, are also excellent candidates for enhancing CTL responses (Table 5).

**Table 5: hTERT HLA-DR peptides predicted by NetMHCII algorithm**

| **Peptide** | **Sequence** | **DR1** | **DR3** | **DR4** | **DR7** | **DR11** | **DR15** | **DRB3** |
|---|---|---|---|---|---|---|---|---|
| TERT 44-58 (UCP1) | PAAFRALVAQCLVCV (SEQ ID NO:1) | **4.5** | 5363.4 | 90.6 | **11.4** | 85.6 | 350 | 127 |
| TERT 578-592 (UCP2) | KSVWSKLQSIGIRQH (SEQ ID NO:2) | **7.1** | 7522.9 | 381.4 | **29.0** | 94.2 | 119.2 | 11328.6 |
| TERT 916-930 (UCP3) | GTAFVQMPAHGLFPW (SEQ ID NO:3) | **3.3** | 972.7 | **46.8** | **28.9** | 94.8 | 215.2 | 1733.2 |
| TERT 1041-1055 (UCP4) | SLCYSILKAKNAGMS (SEQ ID NO:4) | **4.8** | 2485.2 | **59.7** | **38.5** | **12** | 94.5 | 17806.4 |
| SB1 | SFLLSSLRPSLTGAR (SEQ ID NO:5) | **4.4** | **36.1** | 66.8 | 226 | 14.7 | 325.5 | 2962 |
| SB2 | SRPWMPGTPRRLPRL (SEQ ID NO:6) | **9.5** | 14790 | 2113 | **16.1** | 394 | 2758 | 12187 |
| SB3 | RPLFLELLGNHAQCP (SEQ ID NO:7) | **3.9** | 3619 | **19.6** | 587.4 | 67 | 62.6 | 101.35 |
| SB4 | AKFLHWLMSVYVVEL (SEQ ID NO:8) | **4.2** | 5383 | 283.3 | **12.4** | 205 | 27.7 | 1365 |
| SB5 | VKALFSVLNYERARR (SEQ ID NO:9) | **12.0** | 513 | **40.4** | 243 | **26.8** | 88 | 9416 |
| SB6 | ELYFVKVDVTGAYDT (SEQ ID NO:10) | **10.5** | **47.2** | **25.1** | 320 | 124 | 1132 | 11.7 |
| SB7 | LQPYMRQFVAHLQET (SEQ ID NO:11) | **9.7** | 1428 | **40.3** | 236 | 150 | **32.8** | 1428 |
| SB8 | FLRFMCHHAVRIRGK (SEQ ID NO:12) | **9.5** | 1638 | 68.8 | **4.3** | **18.4** | 50.1 | 999 |
| SB9 | AFVQMPAHGLFPWCG (SEQ ID NO:13) | **3.6** | 1512 | 94.7 | 64.4 | 199 | 471 | 3307 |
| SB10 | YSSYARTSIRASLTF (SEQ ID NO:14) | **9.3** | 2596 | **40.3** | 109 | **20.5** | 1020 | 5395 |
| SB11 | NRGFKAGRNMRRKLF (SEQ ID NO:15) | **7.5** | 105.7 | 94.8 | 1642 | 89.7 | 461.9 | 3066 |
| SB12 | NIYKILLLQAYRFHA (SEQ ID NO:16) | **5.7** | 322.2 | 83 | 282.6 | **13.2** | **4.1** | 1088.9 |
| SB13 | PTFFLRVISDTASLC (SEQ ID NO:17) | **4.9** | 160.5 | **10.0** | 69.8 | **34.7** | 208.9 | **45.7** |
| SB14 | RVTYVPLLGSLRTAQ (SEQ ID NO:18) | **6.8** | 279.3 | 72.4 | 1875 | **41.4** | 159.4 | 8867 |
| SB15 | LGSLRTAQTQLSRKL (SEQ ID NO:19) | **5.8** | 285.9 | 259 | 172.1 | 466 | 2100.1 | 11885 |
| SB16 | GTTLTALEAAANPAL (SEQ ID NO:20) | **7.2** | 15029 | **39.1** | 295.6 | 962.9 | 3396 | 10799 |

| | | | | | | | | |
|---|---|---|---|---|---|---|---|---|
| Strong binder (SB) epitope candidates were predicted using the NetMHCII version 2.2 algorithm (Nielsen and Lund, 2009). Sequences are those of wild type hTERT (Acc. Nr NM_198253). The affinity of strong binders is given in nM. Strong binder threshold <50 nM **(in bold).** Weak binder threshold >500 nM. | | | | | | | | |

According to the present invention, the term "CD4 epitope of TERT" encompasses any of the above sequences.

### The CD8 epitopes:

The CD8 epitope encoded by the vectors of the invention is a peptide that is able to activate a CD8 T cell response against an antigen. Preferably, said CD8 epitope is able to activate a CD8 antitumoral response or a CD8 response against a viral, bacterial or parasitic antigen. In a particular embodiment, the vector(s) encode all or part of a viral, bacterial or parasitic antigen which comprises MHC class I epitopes.

The vector(s) which comprise said tumoral, viral, bacterial or parasitic CD8 epitope thus preferably encode a full length, or substantially full, tumoral, viral, bacterial or parasitic CD8 antigen, or CD8 epitope fragments thereof. Most advantageously, the present invention makes use of non-oncogenic mutants of said CD8 antigens. Examples of CD8 epitope peptides derive from the following antigens: tyrosinase, alphafetoprotein, carcinoembryonic antigen (CEA), CA-125, MUC-1, epithelial tumor antigen, Melanoma-associated antigen, P1A, MART-1/Melan-A and gp 100/pMe1l7 as well as tyrosinase-related protein pg75 and MUM-1, HER2/neu, human papillomavirus proteins E6 and E7, survivin, GnT-V, beta-catenin, CDK4, p15, MAGE1, MAGE3, BAGE, GAGE, PSMA, TARP, STEAP, HTLV-1 Tax and WT1.

Examples of CD8 epitope peptides include, but are not limited to: gp100.154, NA17-A.nt38, and Melan-A/MART-1.27, CEA.571, Tyrosinase.368-N, p53.65, Her2/neu.369-377, gp100.209, gp100.280, gp100.476, Tyrosinase.368-D, MAGE-3.271, and Her2/neu.654, gp100.457, Melan-A/MART-1.32, p53.149, p53.264, Sur1M2 and HPV E7.86.

In a particular embodiment, said CD8 epitope is a non-oncogenic mutant of HPV E7 antigen. This antigen shows several class I epitopes and lacks high affinity epitopes to HLA MHC class II determinants. Hence, DNA vaccine approaches targeting only E7 has always given disappointing results (Chen et al, 2000). The present invention enhances the immune response against this antigen, which is particularly useful in treating a cervix cancer.

### Vectors and administration

The expression vectors used in the present invention can provide for expression in *vitro* and/or in *vivo* (e.g. in a suitable host cell, host organism and/or expression system). They typically comprise a polynucleotide sequence as defined above, and regulatory sequences (such as a suitable promoter(s), enhancer(s), terminator(s), etc.) allowing the expression (e.g. transcription and translation) of the protein product in the host cell or host organism.

The vectors according to the invention may be in the form of a vector, such as for example a plasmid, cosmid, YAC, a viral vector or transposon.

In a preferred but non-limiting aspect, a vector of the invention comprises i) at least one nucleic acid as described above; operably connected to ii) one or more regulatory elements, such as a promoter and optionally a suitable terminator; and optionally also iii) one or more further elements of genetic constructs such as 3'- or 5'-UTR sequences, leader sequences, selection markers, expression markers/reporter genes, and/or elements that may facilitate or increase (the efficiency of) transformation or integration.

In particular embodiments, the vector(s) used in the invention may comprise additional inserts encoding molecular adjuvants (cytokines, chemokines or costimulatory molecules), small DNA sequences promoting MHC antigen presentation e.g. IMX313, IMAXIO technology (oligomerization of the antigen), adjuncts that assist antigen to enter specific cell compartment (e.g. LAMP-1) or that may act as adjuvants in stimulating or directing the immune response.

In a particular embodiment, the genetic construct can be prepared by digesting the nucleic acid polymer with a restriction endonuclease and cloning into a plasmid containing a promoter such as the SV40 promoter, the cytomegalovirus (CMV) promoter or the Rous sarcoma virus (RSV) promoter.

Other vectors include retroviral vectors, lentivirus vectors, adenovirus vectors, vaccinia virus vectors, pox virus vectors, measles virus vectors and adenovirus-associated vectors.

In a particular embodiment, a kit is provided, which comprises
a. a first container containing at least a DNA expression vector which encodes said at least two CD4 epitopes of TERT and
b. a second container containing at least a DNA expression vector which encodes at least one tumor, viral, bacterial, or parasitic CD8 epitope.

The kit may further provide at least one additional container containing at least another DNA expression vector which encodes at least a further tumor, viral, bacterial, or parasitic CD8 epitope. Such kits may be particularly useful in a context of a vaccination against a plurality of different tumor, viral, bacterial or parasitic antigens. Such antigens may derive from the same protein, or from different proteins, they may be expressed by the same or different types of tumor, or the same or different viral, bacterial or parasitic agent.

The DNA expression vector which encodes said at least two CD4 epitopes of TERT and the DNA expression vector which encodes at least one tumor, viral, bacterial, or parasitic CD8 epitope, if provided in separate containers, are preferably administered simultaneously, or substantially simultaneously. In a particular embodiment, they are injected topically in close vicinity.

In a particular embodiment, the containers or species can be mixed extemporaneously, or in advance before administration to the subject.

Compositions can be prepared, comprising said vector(s). The compositions are immunogenic. They can comprise a carrier or excipients that are suitable for administration in humans or mammals (i.e. non-toxic, and, if necessary, sterile). Such excipients include liquid, semisolid, or solid diluents that serve as pharmaceutical vehicles, isotonic agents, stabilizers, or any adjuvant. Diluents can include water, saline, dextrose, ethanol, glycerol, and the like. Isotonic agents can include sodium chloride, dextrose, mannitol, sorbitol, and lactose, among others. Stabilizers include albumin, among others. Any adjuvant known in the art may be used in the vaccine composition, including oil-based adjuvants such as Freund's Complete Adjuvant and Freund's Incomplete Adjuvant, mycolate-based adjuvants, bacterial lipopolysaccharide (LPS), peptidoglycans, proteoglycans, aluminum hydroxide, saponin, DEAE-dextran, neutral oils (such as miglyol), vegetable oils (such as arachis oil), Pluronic® polyols.

The vectors or composition can be administered directly or they can be packaged in liposomes or coated onto colloidal gold particles prior to administration. Techniques for packaging DNA vaccines into liposomes are known in the art, for example from Murray, 1991. Similarly, techniques for coating naked DNA onto gold particles are taught in Yang, 1992, and techniques for expression of proteins using viral vectors are found in Adolph, 1996.

The vaccine compositions are preferably administered intradermally, subcutaneously, intramuscularly, into the tumors or in any types of lymphoid organs and are delivered in an amount effective to stimulate an immune response in the host organism. A variety of techniques are available, such as electroporation (e.g. using Cliniporator, IGEA, BTX, Harvard Apparatus), needle-free approaches, such as particle bombardment (e.g., the Pfizer's PMED device) and high-pressure delivery (e.g. Biojector devices, Bioject Medical Technologies), dermal patches (e.g. DermaVir, Genetic Immunity), formulation of DNA vaccine in microparticles or liposomes (e. g. formulation in the lipid compound Vaxfectin, Vical).

In a preferred embodiment administration comprises an electroporation step, also designated herein by the term "electrotransfer", in addition to the injection step (as described in Mir 2008, Sardesai and Weiner 2011). In a most advantageous embodiment, the electrotransfer protocol is as described in international patent application WO2015/063112.

While it will be understood that the amount of material needed will depend on the immunogenicity of each individual construct and cannot be predicted *a priori,* the process of determining the appropriate dosage for any given construct is straightforward. Specifically, a series of dosages of increasing size, starting at about 5 to 30 µg, or preferably 20-25 µg, up to about 500µg to about 5mg, preferably up to 500-1500 µg, 500-1200 µg, or 500-1000 µg, for instance, is administered to the corresponding species and the resulting immune response is observed, for example by detecting the cellular immune response by an IFNγ Elispot assay (as described in the experimental section), by detecting CTL responses using an *in vivo* lysis assay or a chromium release assay or detecting Th (helper T-cell) response using a cytokine release assay.

In a preferred embodiment, the vaccination regimen comprises one to three injections, preferably repeated three or four weeks later.

In a particular embodiment, the vaccination schedule can be composed of one or two injections followed three or four weeks later by at least one cycle of three to five injections. In another embodiment, a primer dose consists of one to three injections, followed by at least a booster dose every year, or every two or years for instance.

These are examples only, and any other vaccination regimen is herein encompassed.

### Therapeutic uses

It is described a method for treating a tumor or an infection in a patient in need thereof, which method comprises administering said patient with the vectors or mixture of vectors described herein.

The vectors used in the present invention especially induce high avidity Th1 polarized CD4 T-cells that significantly enhance CTL responses against weakly immunogenic antigens.

The vectors or mixture of vectors as described above is useful in a method for preventing or treating a tumor in a patient.

A method for preventing or treating a tumor in a patient is described, which method comprises administering an effective amount of said nucleic acid or immunogenic composition in a patient in need thereof. Said nucleic acid or immunogenic composition is administered in an amount sufficient to induce an immune response in the patient.

The tumor may be any undesired proliferation of cells, in particular a benign tumor or a malignant tumor, especially a cancer.

The cancer may be at any stage of development, including the metastatic stage. The cancer may be chronic or non-chronic (acute).

In another embodiment, the disclosure also relates to a vaccine useful in preventing a tumor.

In a particular embodiment, tumor is a solid cancer or a carcinoma. Examples include melanoma, brain tumor such as glioblastoma, neuroblastoma and astrocytoma and carcinomas of the bladder, breast, cervix, colon, lung, especially non-small cell lung cancer (NSCLC), pancreas, prostate, head and neck cancer, or stomach cancer.

In another embodiment, the tumor may be a liquid tumor, e.g. a hematopoietic tumor or leukemia, such as a chronic or acute lymphocytic leukemia, chronic or acute myeloid leukemia, lymphoma including Hodgkin's disease, multiple myeloma, malignant myeloma. Preferably the patient to treat has undergone or is about to undergo a conventional therapy most preferably a first-line conventional therapy.

In a particular embodiment, the treatment may be combined with conventional therapy, including chemotherapy, radiotherapy or surgery. Combinations with adjuvant immunomodulating molecules such as GM-CSF or a cytokine like IL-2 or IL-12, could also be useful.

In another embodiment, the patient may be infected with a virus, a parasite or a bacteria. Examples of virus include papillomavirus, herpes simplex virus, hepatitis virus, adenovirus, myxovirus such as influenza, paramyxovirus, poxvirus such as Vaccinia, lentivirus such as HIV.

The Examples and Figures illustrate the invention without limiting its scope.

### EXAMPLES

### EXAMPLE 1: Induction of a response against the E7 antigen of HPV16 after co-delivery of DNA plasmids

Universal hTERT CD4 epitopes encoded by various DNA constructs were shown to play a helper role in the induction of a CTL response against the E7 antigen of HPV16 after co-delivery of DNA plasmids by intradermal injection combined with electroporation. HLA-A2/DR1 transgenic mice were immunized with DNA constructs encoding hTERT-derived CD4 epitopes (namely UCP for Universal Cancer Peptides) in the presence of a plasmid encoding a non-oncogenic mutant of HPV16 E7 antigen. Ten days after a second immunization with plasmids (boost) the frequency of CD4 and CD8 T-cell immune responses were evaluated in the spleen of animals by an IFN-γ ELISpot assay. Functionally-polarized T cell subsets were identified based on their distinctive patterns of cytokine secretion using a CBA assay.

### Abbreviations

HLA-A2/DR1: HLA-A^{∗}0201/HLA-DRB1^{∗}0101 transgenic, H2 class I/class II KO mice, CBA: Cytometric Bead Array, CTL: Cytotoxic T Lymphocyte, DNA: Deoxyribonucleic acid, EP: Electroporation, HLA: Human Leukocyte Antigen, HPV16: Human Papillomavirus type 16, hTERT: human TERT, ID: Intradermal, IL: Interleukin, IFN-γ: Interferon gamma, MHC: Major Histocompatibility Complex, TAA: Tumor-Associated Antigen, TERT: Telomerase Reverse Transcriptase, TNF-α: tumor necrosis factor alpha, RT: Room temperature, wt: wild type, UCP: Universal Cancer Peptide

### Materials and methods

### Plasmid DNA vectors

### pUCPbasic

pUCPbasic is a 5717 bp plasmid expression vector encoding human telomerase reverse transcriptase (hTERT) derived class II epitopes, namely UCP1, UCP2, UCP3 and UCP4 (101 AA), linked with a 14 AA V5 tag (Figure 1, Figure 8), corresponding to a polypeptide of approximately 12.7 kDa of molecular weight. The hTERT insert sequence is composed of four fragments: AA 2-26, AA 27-51, AA 52-76, AA 77-101 corresponding respectively to wild type hTERT (Acc. Nr NM_198253): AA 39-63 hTERT, AA 573-597 hTERT, AA 911-935 hTERT and AA 1036-1060 hTERT, which includes UCP-1-4 class II epitopes and their 5AA flanking sequences. These fragments contain four well caracterized 15-mer CD4+ hTERT epitopes (TERT 44-58 PAAFRALVAQCLVCV (SEQ ID NO:1), TERT 578-592 KSVWSKLQSIGIRQH (SEQ ID NO:2), TERT 916-930 GTAFVQMPAHGLFPW (SEQ ID NO:3), TERT 1041-1055 SLCYSILKAKNAGMS, (SEQ ID NO:4) and two 9-mer internal CD8+ telomerase epitopes (Godet et al. 2012; Suso et al. 2011).

### pUCP2(4x)

pUCP2(4x) is a 5717 bp plasmid expression vector encoding the human telomerase reverse transcriptase CD4+ 578-592 (KSVWSKLQSIGIRQH, (SEQ ID NO:2), Acc. Nr NM_198253) epitope with 5AA flanking sequences on each side. This UCP2 epitope unit is repeated four times and linked with a 14 AA V5 epitope tag (Figure 2, Figure 9), together corresponding to a polypeptide of approximately 12.7 kDa of molecular weight.

### pDE7

pDE7 is a 5708 bp plasmid expression vector encoding a non-oncogenic HPV16 E7 variant (98 AA) (Wieking et al., 2012) linked with a 14 AA V5 epitope tag (Figure 3, Figure 10), corresponding to a protein of approximately 14-15 kDa of molecular weight. To decrease the risk associated with the delivery of oncogenic HPV E7, four known oncogenic regions within E7 were mutated (Munger et al., 2004). Mutations introduced in E7 prevent its ability to bind/inactivate pRb and to associate with Mi2β that enhances cell growth (Brehm et al., 1999).

### Gene synthesis and cloning

Genes were synthesized through an overlapping 40-mer oligonucleotides assembly process (GeneCust, Luxembourg) and included unique flanking restriction sites *Hind*III/*Xho*I. The synthetic genes were cloned between *Hind*III and *Xho*I restriction sites of the pcDNA3.1(+) expression vector (Life Technologies, Carlsbad, USA). pcDNA3.1(+) is a 5.4 kb vector derived from pcDNA3.0 which was designed for high-level of stable and transient expressions in mammalian cells. This vector contains the human cytomegalovirus immediate-early (CMV-IE) promoter and the bovine growth hormone polyadenylation (BHG-polyA) signal as termination sequence.

### Endotoxin free plasmid production

Plasmids were transformed and produced in *E. coli* 5-alpha cells (fhuA2Δ(argF-lacZ)U169 phoA glnV44 Φ80 Δ(lacZ)M15 gyrA96 recA1 relA1 endA1 thi-1 hsdR17) (Lucigen Corporation, Middleton, USA, ref. 60602-2) by RD Biotech (Besançon, France). Endotoxin-free plasmids were resuspended in 1x sterile PBS at 4 mg/mL. The constructs were verified by restriction enzyme digestion.

### Cell culture and transient transfection

HEK293T cells were cultured in Dulbecco's modified Eagle's medium (DMEM) supplemented with 10% heat-inactivated fetal calf serum (PAA, Velizy-Villacoublay, France) and 1% penicillin/streptomycin (Life technologies SAS, Saint-Aubin, France).

Cells were grown as monolayers in 75 cm² flasks at 37°C in a humidified atmosphere containing 5% CO₂. 8x10⁵ cells were seeded in six-well tissue culture plates and incubated for 24 h. The cells were grown to 70-80% confluence on the day of transfection.

pUCPbasic, pUCP2(4x), pDE7 constructs were transfected into target cells using jetPrime cationic polymer transfection reagent (Polyplus-transfection Inc., Illkirch, France). pcDNA3.1 plasmid transfected cells were used as negative control. After 48 hours of transfection, cells were harvested and analyzed for expression.

### Western blot

pUCPbasic, pUCP2(4x), pDE7 and pcDNA3.1 transfected HEK293T cells were lysed on ice for 20 minutes in RIPA buffer (Sigma Aldrich SARL, Saint-Quentin Fallavier, France) supplemented with a protease inhibitor cocktail (Roche Diagnostic, Indianapolis, USA). Lysates were cleared by centrifugation at 14,000 rpm for 15 minutes at 4°C. Supernatants were harvested and the protein concentration was measured using the Bradford colorimetric assay.

The pDE7 protein was immunoprecipitated using anti-V5 conjugated agarose beads (Abcam, Cambridge, UK). Cells were lysed with a cell lysis buffer containing 125 mM NaCl, 50 mM Tris pH8, 25 mM EDTA pH8 and 0.5% NP40 for 15 min and centrifuged at 1600 rpm for 5 min, and then 20 µl of anti-V5 agarose (Abcam) was added. Samples were rotated overnight at 4°C and washed three times with cold cell lysis buffer.

All protein samples were resuspended in 1x sample buffer (Life technologies SAS, Saint-Aubin, France), denatured 5 minutes at 90°C, separated on Nu-PAGE® Novex 4-12% Bis-Tris gels (Life Technologies) and electroblotted onto PVDF membranes (iBlot® transfer stack, Life Technologies) using the iBlot® device (Life Technologies). The ProSieve™ QuadColor™ protein marker (Lonza, Levallois-Perret, France) was used to determine molecular weight. The membranes were cut approximately at 40 kDa and blocked with PBS IX, 0.05% Tween®20, 3% milk. The lower part of the membrane was probed with an anti-V5 mouse monoclonal antibody (Life Technologies) diluted at 1/5000 and the upper part was probed with an anti-β-actin mouse monoclonal antibody (Sigma Aldrich SARL, Saint-Quentin Fallavier, France) diluted at 1/5000. The membranes were then incubated with an anti-mouse HRP linked antibody (GE Healthcare, Vélizy, France) diluted at 1/5000 and the proteins were detected by enhanced chemiluminescence assay using ECL HRP chemiluminescent substrate Reagent Kit. Membranes were visualized on a ChemiDoc XRS system (Bio-Rad, Marnes-la-Coquette, France) and analyzed using Image Lab 5.2.1 (Bio-Rad) software.

### Mice

Transgenic HLA-A^{∗}0201/DRB1^{∗}0101 (HLA-A2/DR1) mice (13-16 week old) were supplied by Institut Pasteur (Paris, France) or CDTA-TAAM (Orleans, France). These mice express the human HLA-A^{∗}0201 α1α2 domains and the murine α3 domain of the H2-D molecule, the human β2-microglobulin, and HLA-DRB1^{∗}0101 and HLA-DRA^{∗}0101 molecules. They are knock-out for murine H2-D^{b}, H2-K^{b} and IA^{b} genes (Pajot et al., 2004).

Animals were housed at the Institut Pasteur's pathogen-free animal facility. All animals were handled in strict accordance with good animal practice and complied with local animal experimentation (Directive 2010/63/UE).

### Immunization

Prior to treatment, mice were anesthetized with a mix solution of xylazine 2% (Rompun, Bayer Santé, Loos, France) and ketamine 8% (Imalgen 1000, Merial, Lyon, France) in PBS (Life technologies SAS, Saint-Aubin, France) through the intraperitoneal route (IP) according to individual animal weight and duration of anesthesia. Plasmids were then injected intradermally (ID) on the lower back at day 0 (prime) and at day 21 (boost) and electroporated. Briefly, immediately after ID injection, a skin fold was made at the injection site, entirely covered with conductive gel and placed between the electrode plates of the electroporation device (CLINIPORATOR® 2, IGEA, Carpi, Italy). Two pulses of different voltages were applied (HV-LV) as described below. The experimental groups were defined as summarized in Table 6.

**Table 6: Repartition of mice-numbering and treatment conditions**

| **Groups** | **Number animals** | **Mouse number** | **Treatment** | **Dose (µg)** | **Volume of injection/ mouse** | **Route** | **Electroporation parameters** |
|---|---|---|---|---|---|---|---|
| 1 | 4 | 1-4 | pDE7/pcDNA3.1 | 100+100 | 50 µl | ID | Electrode: |
| 2 | 5 | 5-9 | pDE7/pUCP2(4x) | 100+100 | | | P-30-8B (0.5 cm apart) |
| 3 | 4 | 10-13 | pDE7/pUCPbasic | 100+100 | | | **HV= 1250 V/cm, 1 Hz, 100 µs, 1 pulse, 1000 ms break LV= 180 V/cm, 1 Hz, 400 ms, 1 pulse** |

| | | | | | | | |
|---|---|---|---|---|---|---|---|
| ID=intradermal; | | | | | | | |

### Peptides

For immune response analyses, hTERT peptides restricted to HLA-DR and E7 peptides restricted to HLA-A^{∗}0201 were previously described or were determined by *in silico* epitope prediction using SYFPEITHI (www.syfpeithi.de) Rammensee et al, 1999) or NetMHCII 2.2 (Nielsen and Lund, 2009) algorithms available online. All synthetic peptides were purchased lyophilized (>90% purity) from Proimmune (Oxford, United Kingdom). Lyophilized peptides were dissolved in sterile water at 2 mg/mL and stored at -80°C prior use. Details of peptide sequences and MHC restrictions are shown in Table 7.

**Table 7: MHC restricted hTERT and E7 peptides**

| **Peptide** | **Pool** | **Sequence** | **MHC** |
|---|---|---|---|
| TERT 44-58 (UCP1) | Pool 1 | PAAFRALVAQCLVCV (SEQ ID NO:1) | HLA-DR |
| TERT 578-592 (UCP2) | | KSVWSKLQSIGIRQH (SEQ ID NO:2) | |
| TERT 916-930 (UCP3) | | GTAFVQMPAHGLFPW (SEQ ID NO:3) | |
| TERT 1041-1055 (UCP4) | | SLCYSILKAKNAGMS (SEQ ID NO:4) | |
| E7 9-23 | Pool 2 | HEYMLDLQPETTDLY (SEQ ID NO:27) | HLA-DRB1^{∗}0101 |
| E7 73-87 | | HVDIRTLEDLLMGTL (SEQ ID NO:28) | |
| E7 7-15 | Pool 3 | TLHEYMLDL (SEQ ID NO:29) | HLA-A^{∗}0201 |
| E7 11-20 | | YMLDLQPETT (SEQ ID NO:30) | |
| E7 14-22 | | DLQPETTDL (SEQ ID NO:31) | |
| E7 78-87 | | TLEDLLMGTL (SEQ ID NO:32) | |
| E7 81-90 | | DLLMGTLGIV (SEQ ID NO:33) | |
| E7 82-90 | | LLMGTLGIV (SEQ ID NO:34) | |
| E7 86-93 | | TLGIVCPI (SEQ ID NO:35) | |

### IFN-γ ELISpot assay

Ten days after the boost vaccination, mice were euthanized by CO₂ narcosis and spleens were harvested. Spleens were then mashed and splenocyte suspensions were filtered through a 70 µm nylon mesh (Cell Strainer, BD Biosciences, Pont-de-Claix, France), purified on ficoll (Lymphocyte Separation Medium, Eurobio, Les Ulis, France) and numerated using the Cellometer® Auto T4 Plus counter (Ozyme, Montigny-le-Bretonneux, France). Purified splenocytes were then added to ELISpot PVDF microplates (IFN-γ ELISpot kit, Diaclone, Besançon, France) coated with an anti-mouse IFN-γ antibody at 2×10⁵ cells/well in triplicates and stimulated with 5 µg/mL of peptides (see Table 7), 10 µg/mL PMA-ionomycin (Sigma-Aldrich), or mock stimulated with serum free culture medium. To score the number of hTERT and E7 antigen-specific IFN-γ secreting cells, three different peptide pools were used: CD4+ hTERT (pool 1), CD4 E7 (pool 2) or CD8 E7 (pool 3) (Table 7). After 19 hours, spots were revealed with a biotin-conjugated anti-IFNγ detection antibody followed by streptavidin-HRP and BCIP/NBT substrate solution. Spots were counted and analyzed using the ImmunoSpot® ELISpot counter and software (Cellular Technology Limited, Bonn, Germany).

### Cytokine Binding Assay (CBA)

Splenocytes (6x10⁵ cells) from vaccinated HLA-A2/DR1 mice were cultured for 24 h at 37°C with HLA-DR-restricted hTERT derived peptides from pool 1 at a final concentration of 5 µg/mL. Cytokine culture supernatants were recovered and kept frozen at -20°C until testing. Mouse Th1/Th2/Th17 Cytometric Beads Array (CBA, BD Biosciences) kit was used to quantify respectively the concentration of IL-2, IFN-γ, TNF-α, IL-4, IL-6, IL-10 and IL-17a. The CBA immunoassay was carried out according to the manufacturer's instructions. Flow cytometry was performed using the FACScan LSRII flow cytometer (BD Biosciences). Quantitative results were generated using the FCAP Array TM Software version 3.0 (Becton Dickinson, Pont-de-Claix, France).

### Statistical analysis

Differences between two groups were evaluated by unpaired t-test or non parametric Mann and Whitney *U* test. The Pearson's correlation coefficient test was used to evaluate the correlation between hTERT class II and E7 class I responses in immunized mice. All computer analyses were performed using GraphPad Prism 6 (GraphPad Software Inc. La Jolla, CA, USA). p values < 0.05 were considered statistically significant.

### Results

### Characterization and sequence analysis of constructs

DNA transgenes expressing UCPbasic, UCP2(4x) and DE7 protein were synthesized and cloned into pcDNA3.1(+) Life Technologies expression vector as shown by restriction enzyme digestion and electrophoresis (Figure 4). Inserts and junctions were sequenced using T7 (forward 5' TAATACGACTCACTATAGGG 3', (SEQ ID NO:36)) and/or BGH (reverse 5'TAGAAGGCACAGTCGAGG 3' (SEQ ID NO:37)) primers matching the vector sequence flanking the DNA insert (see Table 8). Sequencing results confirmed that transgenes have been correctly synthesized and cloned.

**Table 8: Characterization of constructs based on restriction mapping and DNA sequencing**

| **Plasmid** | **Restriction enzyme or sequencing primer** | **Sequence length (bp)** |
|---|---|---|
| pUCPbasic | HindIII/XbaI | 369+5348* |
| pUCP2(4x) | HindIII/XhoI | 363+5354* |
| pDE7 | HindIII/XhoI | 354+5354* |
| pUCPbasic | BGH primer | HindIII/XhoI sequence confirmed (911-1294 bp) |
| pUCP2(4x) | BGH primer | HindIII/XhoI sequence confirmed (911-1320 bp) |
| pDE7 | T7 and BGH primers | HindIII/XhoI sequence confirmed (911-1364 bp) |

| | | |
|---|---|---|
| * Digestion product sizes (bp) and generated fragments | | |

### In vitro transgene expression

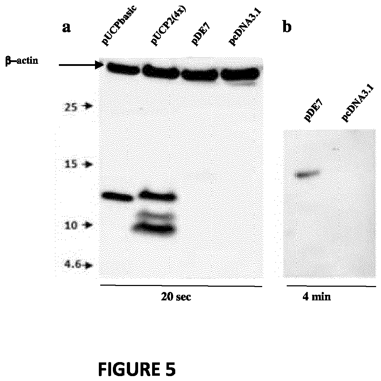

The expression of pUCPbasic and pUCP2(4x) polypeptides, as well as pDE7 protein was assessed by western blot assay. The constructs were transiently transfected into HEK293T cell line, which were then cultured for 48 hours. pUCPbasic and pUCP2(4x) polypeptides were identified at a molecular weight of approximately 12.6 and 12.8 kDa, respectively. Two additional bands were observed for UCP2(4x) (about 11 and 9 kDa) which may be accounted for protein degradation (Figure 5a). For unknown reasons, it was difficult to detect the DE7 protein directly by western blot (Figure 5a). An immunoprecipitation step using anti-V5 conjugated agarose beads was necessary to achieve this goal (Figure 5b). The pDE7 protein was detected at the expected molecular weight of about 14.5 kDa. pcDNA3.1 transfected cells were used as negative control. These results validate *in vitro* expression pattern and identity of pUCPbasic, pUCP2(4x) and pDE7 DNA constructs.

### hTERT helper epitopes increase E7-specific immune responses in HLA transgenic mice

In order to determine the frequency of hTERT CD4+ and E7 CD8+/CD4+ specific T-cells in HLA-A2/DR1 transgenic mice immunized with pUCPbasic, pUCP2(4x) and pDE7, ELISpot IFN-γ analyses were performed.

As shown in Figure 6a, immunization with pUCPbasic or pUCP2(4x) induced a strong hTERT CD4+ specific cellular immune response. As expected, no E7 CD4+ specific immune response was detected (Figure 6b) as the E7 protein does not present high affinity class II epitopes. Co-immunization of pUCPbasic or pUCP2(4x) with pDE7 showed a significant E7 CD8+ specific immune response (Figure 6c). When compared to the pDE7+pcDNA3.1 DNA vaccine, pDE7 co-immunized with pUCPbasic or pUCP2(4x) was potent in driving an E7-specific immune response, suggesting that pUCPbasic and pUCP2(4x) constructs were able to significantly increase the magnitude of CD8+ cellular immune responses against the E7 antigen.

A positive correlation between the strength of the hTERT CD4+ class II and E7 CD8+ class I specific immune responses was observed, both individually (Figure 6a, c, see numbered points corresponding to the animal identification number) and when aggregated (Figure 6d).

Thus, the increased level of CD4 T cell response was directly proportional to the magnitude of CD8 T cell expansion for each animal. Data analysis using the Pearson's correlation coefficient test indicated that the strength of correlation between the CD4+ and CD8+ responses was very high (R² = 0.95) and statistically significant (p=0.005) at least for pUCP2(4x) (Figure 6e). This analysis was less reliable for pUCPbasic (R²=0.42, p=0.35) probably because of the low number of animals included in this study (Figure 6f).

Finally, in order to evaluate the role of Th1/Th2 immune response, the different cytokines secreted by hTERT specific CD4 T-cells were examined. To this aim, splenocytes of vaccinated transgenic mice from a second independent experiment were stimulated *in vitro* for 24 hours with a pool of hTERT peptides or left unstimulated. Supernatants were recovered and assayed in a Cytokine Binding Assay (CBA). Elevated concentrations of Th1 cytokines IFN-γ, TNF-α, IL-2 and Th2 IL-6, but not IL-4, IL-10 and IL-17 were detected in culture supernatants of splenocytes recovered from mice vaccinated with pUCPbasic and pUCP2(4x) in comparison with those of control mice (pDE7 + pcDNA3.1 and pcDNA3.1) (Figure 7). This result indicates that UCP immunization preferentially induced a Th1-polarized immune response *in vivo.*

Thus, vaccination with pUCPbasic or pUCP2(4x) induces a specific Th1 polarized CD4 T-cell response and is able to promote the expansion of E7 specific CD8 T-cells *in vivo.*

### Conclusion

The results showed that hTERT CD4 polyepitope DNA constructs were correctly processed and presented to CD4 T cells in HLA-A2/DR1 transgenic mice in the context of a HLA class II restriction and could generate efficient CD4 Th1 cells that produce mainly IFN-γ, TNF-α and IL-2. Moreover, hTERT CD4 Th1 cell response is highly correlated with CD8 T-cell response against the E7 antigen and strongly improves the CD8 T-cell immune response against E7 class I-restricted epitopes and thus the efficacy of E7 antigen vaccination.

These results confirm that it is possible to increase the strength and the quality of the immune response against poorly immunogenic antigens by complementing the vaccine formulation by CD4 Th1 epitopes through a DNA delivery approach.

### REFERENCES

- Adolph, K. 1996 ed. "Viral Genome Methods" CRC Press, Florida
- Bernhardt SL, Gjertsen MK, Trachsel S, Moller M, Eriksen JA, et al. (2006) Telomerase peptide vaccination of patients with non-resectable pancreatic cancer: A dose escalating phase I/II study. Br J Cancer 95: 1474-1482.
- Bolonaki I, Kotsakis A, Papadimitraki E, Aggouraki D, Konsolakis G, et al. (2007) Vaccination of patients with advanced non-small-cell lung cancer with an optimized cryptic human telomerase reverse transcriptase peptide. J Clin Oncol 25: 2727-2734.
- Brehm A, Nielsen SJ, Miska EA, McCance DJ, Reid JL, et al. (1999) The E7 oncoprotein associates with Mi2 and histone deacetylase activity to promote cell growth. EMBO J 18: 2449-2458.
- Chen CH, Wang TL, Hung CF, Yang Y, Young RA, et al. (2000) Enhancement of DNA vaccine potency by linkage of antigen gene to an HSP70 gene. Cancer Res 60: 1035-1042.
- Cheever MA, Allison JP, Ferris AS, Finn OJ, Hastings BM, et al. (2009) The prioritization of cancer antigens: a national cancer institute pilot project for the acceleration of translational research. Clin Cancer Res 15: 5323-5337.
- Godet Y, Fabre E, Dosset M, Lamuraglia M, Levionnois E, et al. (2012) Analysis of spontaneous tumor-specific CD4 T-cell immunity in lung cancer using promiscuous HLA-DR telomerase-derived epitopes: potential synergistic effect with chemotherapy response. Clin Cancer Res 18: 2943-2953.
- Mir LM. 2008. Application of electroporation gene therapy: past, current, and future. Methods Mol Biol 423: 3-17.
- Munger K, Baldwin A, Edwards KM, Hayakawa H, Nguyen CL, et al. (2004) Mechanisms of human papillomavirus-induced oncogenesis. J Virol 78: 11451-11460.
- Murray, 1991, ed. "Gene Transfer and Expression Protocols" Humana Pres, Clifton, N.J.
- Nielsen M, Lund O (2009) NN-align. An artificial neural network-based alignment algorithm for MHC class II peptide binding prediction. BMC Bioinformatics 10: 296.
- Pajot A, Michel ML, Fazilleau N, Pancre V, Auriault C, et al. (2004) A mouse model of human adaptive immune functions: HLA-A2.1-/HLA-DR1-transgenic H-2 class I-/class II-knockout mice. Eur J Immunol 34: 3060-3069.
- Rammensee H, Bachmann J, Emmerich NP, Bachor OA, Stevanovic S (1999) SYFPEITHI: database for MHC ligands and peptide motifs. Immunogenetics 50: 213-219.
- Sardesai NY, Weiner DB. 2011. Electroporation delivery of DNA vaccines: prospects for success. Curr Opin Immunol 23: 421-429.
- Shay JW, Bacchetti S (1997) A survey of telomerase activity in human cancer. Eur J Cancer 33: 787-791.
- Shedlock DJ, Shen H (2003) Requirement for CD4 T cell help in generating functional CD8 T cell memory. Science 300: 337-339.
- Suso EM, Dueland S, Rasmussen AM, Vetrhus T, Aamdal S, et al. (2011) hTERT mRNA dendritic cell vaccination: complete response in a pancreatic cancer patient associated with response against several hTERT epitopes. Cancer Immunol Immunother 60: 809-818.
- Wieking BG, Vermeer DW, Spanos WC, Lee KM, Vermeer P, et al. (2012) A non-oncogenic HPV 16 E6/E7 vaccine enhances treatment of HPV expressing tumors. Cancer Gene Ther 19: 667-674.
- Yang, 1992, "Gene transfer into mammalian somatic cells in vivo", Crit. Rev. Biotech. 12: 335-356

## Claims

1. A DNA expression vector or a mixture of DNA expression vectors which encodes at least two CD4 epitopes of telomerase reverse transcriptase (TERT) and at least one tumor, viral, bacterial, or parasitic CD8 epitope; wherein said at least two CD4 epitopes of TERT are identical, and repeated.

2. The mixture of DNA expression vectors of claim 1, comprising i) at least a DNA expression vector which encodes said at least two CD4 epitopes of TERT and ii) at least a DNA expression vector which encodes said at least one tumor, viral, bacterial, or parasitic CD8 epitope.

3. The DNA expression vector of claim 1, which encodes said at least two CD4 epitopes of TERT and said tumor, viral, bacterial, or parasitic CD8 epitope.

4. The DNA expression vector or a mixture of DNA expression vectors according to any of claim 1 to 3, wherein said TERT is human TERT.

5. The DNA expression vector or a mixture of DNA expression vectors according to any of claim 1 to 4, which encodes between 4 and 10, preferably between 4 and 8, of said identical CD4 epitopes of TERT.

6. The DNA expression vector or a mixture of DNA expression vectors according to any of claim 1 to 5, wherein at least one of said CD4 epitopes of TERT is selected from the group consisting of SEQ ID NO:1, SEQ ID NO:2, SEQ ID NO:3, and SEQ ID NO:4, preferably wherein the vector or mixture of vectors encodes a polyepitopic sequence comprising all of SEQ ID NO:1, SEQ ID NO:2, SEQ ID NO:3, and SEQ ID NO:4.

7. The DNA expression vector or a mixture of DNA expression vectors according to any of claim 6, which encodes a repetition at least two, preferably at least four, epitopes of SEQ ID NO:2.

8. The DNA expression vector or a mixture of DNA expression vectors according to any of claim 1 to 7, which encode, as said CD8 epitope, all or part of a viral, bacterial or parasitic antigen which comprises MHC class I epitopes, preferably a non-oncogenic mutant of HPV E7 antigen.

9. A kit comprising
a. a first container containing at least a DNA expression vector which encodes said at least two CD4 epitopes of TERT; wherein said at least two CD4 epitopes of TERT are identical, and repeated, and
b. a second container containing at least a DNA expression vector which encodes a tumor, viral, bacterial, or parasitic CD8 epitope.

10. The kit of claim 9, further comprising at least an additional container containing at least a DNA expression vector which encodes a further distinct tumor, viral, bacterial, or parasitic CD8 epitope.

11. The DNA expression vector or a mixture of DNA expression vectors according to any of claim 1 to 8, or the kit of claim 9 or 10, for use in treating a tumor or an infection in a patient.

12. The DNA expression vector, mixture of DNA expression vectors, or the kit for use according to claim 11, wherein the tumor is a cancer, such as a cancer selected from the group consisting of chronic lymphocytic leukemia, chronic myeloid leukemia, multiple myeloma, malignant myeloma, Hodgkin's disease, melanoma, brain tumor such as glioblastoma, neuroblastoma and astrocytoma and carcinomas of the bladder, breast, cervix, colon, lung, pancreas, prostate, head and neck, or stomach, preferably wherein the tumor is a cancer induced by a virus.

13. The DNA expression vector, mixture of DNA expression vectors or the kit, for use according to claim 11 or 12, administrated by intradermal injection, preferably combined with electroporation.

## Patentansprüche

1. Ein DNA-Expressionsvektor oder ein Gemisch von DNA-Expressionsvektoren, codierend wenigstens zwei CD4-Epitope von Telomerase-Reverse-Transkriptase (TERT) und wenigstens ein Tumor-, virales, bakterielles oder parasitisches CD8-Epitop, wobei besagte wenigstens zwei CD4-Epitope von TERT identisch und wiederholt sind.

2. Das Gemisch von DNA-Expressionsvektoren nach Anspruch 1, umfassend i) wenigstens einen DNA-Expressionsvektor, der besagte wenigstens zwei CD4-Epitope von TERT codiert, und ii) wenigstens einen DNA-Expressionsvektor, der besagtes wenigstens eine Tumor-, virales, bakterielles oder parasitisches CD8-Epitop codiert.

3. Der DNA-Expressionsvektor nach Anspruch 1, der besagte wenigstens zwei CD4-Epitope von TERT und besagtes Tumor-, virales, bakterielles oder parasitisches CD8-Epitop codiert.

4. Der DNA-Expressionsvektor oder ein Gemisch von DNA-Expressionsvektoren gemäß einem der Ansprüche 1 bis 3, wobei besagte TERT humane TERT ist.

5. Der DNA-Expressionsvektor oder ein Gemisch von DNA-Expressionsvektoren gemäß einem der Ansprüche 1 bis 4, codierend zwischen 4 und 10, vorzugsweise zwischen 4 und 8 besagter identischer CD4-Epitope von TERT.

6. Der DNA-Expressionsvektor oder ein Gemisch von DNA-Expressionsvektoren gemäß einem der Ansprüche 1 bis 5, wobei wenigstens eines besagter CD4-Epitope von TERT aus der Gruppe ausgewählt ist, bestehend aus SEQ ID NO: 1, SEQ ID NO: 2, SEQ ID NO: 3 und SEQ ID NO: 4, wobei vorzugsweise der Vektor oder das Gemisch von Vektoren eine Polyepitop-Sequenz codiert, umfassend alle Sequenzen von SEQ ID NO: 1, SEQ ID NO: 2, SEQ ID NO: 3 und SEQ ID NO: 4.

7. Der DNA-Expressionsvektor oder ein Gemisch von DNA-Expressionsvektoren gemäß Anspruch 6, codierend eine Wiederholung von wenigstens zwei, vorzugsweise wenigstens vier Epitopen von SEQ ID NO: 2.

8. Der DNA-Expressionsvektor oder ein Gemisch von DNA-Expressionsvektoren gemäß einem der Ansprüche 1 bis 7, codierend als besagtes CD8-Epitop das gesamte oder einen Teil eines viralen, bakteriellen oder parasitischen Antigens, welches MHC Klasse I-Epitope, vorzugsweise eine nicht-onkogene Mutante des HPV-E7-Antigens umfasst.

9. Ein Kit umfassend
a) einen ersten Behälter, enthaltend wenigstens einen DNA-Expressionsvektor, der besagte wenigstens zwei CD4-Epitope von TERT codiert, wobei besagte wenigstens zwei CD4-Epitope von TERT identisch und wiederholt sind, und
b) einen zweiten Behälter, enthaltend wenigstens einen DNA-Expressionsvektor, der ein Tumor-, virales, bakterielles oder parasitisches CD8-Epitop codiert.

10. Das Kit nach Anspruch 9, außerdem umfassend wenigstens einen weiteren Behälter, enthaltend wenigstens einen DNA-Expressionsvektor, der ein anderes Tumor-, virales, bakterielles oder parasitisches CD8-Epitop codiert.

11. Der DNA-Expressionsvektor oder ein Gemisch von DNA-Expressionsvektoren gemäß einem der Ansprüche 1 bis 8 oder das Kit nach Anspruch 9 oder 10 zur Verwendung in der Behandlung eines Tumors oder einer Infektion in einem Patienten.

12. Der DNA-Expressionsvektor, ein Gemisch von DNA-Expressionsvektoren oder das Kit zur Verwendung nach Anspruch 11, wobei der Tumor ein Krebs ist, wie beispielsweise ein Krebs, ausgewählt aus der Gruppe, bestehend aus chronischer lymphatischer Leukämie, chronischer myeloischer Leukämie, multiplem Myelom, malignem Myelom, Morbus Hodgkin, Melanom, Hirntumor, wie Glioblastom, Neuroblastom und Astrozytom, und Karzinomen von Blase, Brust, Gebärmutterhals, Dickdarm, Lunge, Pankreas, Prostata, Kopf und Hals, oder Magen, wobei vorzugsweise der Tumor ein virusinduzierter Krebs ist.

13. Der DNA-Expressionsvektor, ein Gemisch von DNA-Expressionsvektoren oder das Kit zur Verwendung nach Anspruch 11 oder 12, verabreicht mittels intradermaler Injektion, vorzugsweise kombiniert mit Elektroporation.

## Revendications

1. Vecteur d'expression d'ADN ou mélange de vecteurs d'expression d'ADN qui code au moins deux épitopes de CD4 de transcriptase inverse télomérase (TERT) et au moins un épitope de CD8 tumoral, viral, bactérien ou parasitaire ; dans lequel lesdits au moins deux épitopes de CD4 de TERT sont identiques, et répétés.

2. Mélange de vecteurs d'expression d'ADN selon la revendication 1, comprenant i) au moins un vecteur d'expression d'ADN qui code lesdits au moins deux épitopes de CD4 de TERT et ii) au moins un vecteur d'expression d'ADN qui code ledit au moins un épitope de CD8 tumoral, viral, bactérien ou parasitaire.

3. Vecteur d'expression d'ADN selon la revendication 1, qui code lesdits au moins deux épitopes de CD4 de TERT et ledit épitope de CD8 tumoral, viral, bactérien ou parasitaire.

4. Vecteur d'expression d'ADN ou mélange de vecteurs d'expression d'ADN selon l'une quelconque des revendications 1 à 3, dans lequel ladite TERT est une TERT humaine.

5. Vecteur d'expression d'ADN ou mélange de vecteurs d'expression d'ADN selon l'une quelconque des revendications 1 à 4, qui code entre 4 et 10, de préférence entre 4 et 8, desdits épitopes de CD4 identiques de TERT.

6. Vecteur d'expression d'ADN ou mélange de vecteurs d'expression d'ADN selon l'une quelconque des revendications 1 à 5, dans lequel au moins l'un desdits épitopes de CD4 de TERT est choisi dans le groupe constitué par la SEQ ID NO : 1, la SEQ ID NO : 2, la SEQ ID NO : 3 et la SEQ ID NO : 4, de préférence lequel vecteur ou mélange de vecteurs code une séquence polyépitopique comprenant toutes parmi la SEQ ID NO : 1, la SEQ ID NO : 2, la SEQ ID NO : 3, et la SEQ ID NO : 4.

7. Vecteur d'expression d'ADN ou mélange de vecteurs d'expression d'ADN selon la revendication 6, qui code une répétition d'au moins deux, de préférence d'au moins quatre, épitopes de la SEQ ID NO : 2.

8. Vecteur d'expression d'ADN ou mélange de vecteurs d'expression d'ADN selon l'une quelconque des revendications 1 à 7, qui codent, à titre dudit épitope de CD8, tout ou partie d'un antigène viral, bactérien ou parasitaire qui comprend des épitopes de MHC de classe I, de préférence un mutant non oncogène d'antigène E7 de HPV.

9. Kit comprenant
a. un premier récipient contenant au moins un vecteur d'expression d'ADN qui code lesdits au moins deux épitopes de CD4 de TERT ; lesquels au moins deux épitopes de CD4 de TERT sont identiques, et répétés, et
b. un deuxième récipient contenant au moins un vecteur d'expression d'ADN qui code un épitope de CD8 tumoral, viral, bactérien ou parasitaire.

10. Kit selon la revendication 9, comprenant en outre au moins un récipient additionnel contenant au moins un vecteur d'expression d'ADN qui code un autre épitope de CD8 tumoral, viral, bactérien ou parasitaire distinct.

11. Vecteur d'expression d'ADN ou mélange de vecteurs d'expression d'ADN selon l'une quelconque des revendications 1 à 8, ou kit selon la revendication 9 ou 10, pour une utilisation dans le traitement d'une tumeur ou d'une infection chez un patient.

12. Vecteur d'expression d'ADN, mélange de vecteurs d'expression d'ADN, ou kit pour une utilisation selon la revendication 11, dans lequel la tumeur est un cancer, tel qu'un cancer choisi dans le groupe constitué par une leucémie lymphoïde chronique, une leucémie myéloïde chronique, un myélome multiple, un myélome malin, une maladie de Hodgkin, un mélanome, une tumeur cérébrale telle qu'un glioblastome, un neuroblastome et un astrocytome et les carcinomes de la vessie, du sein, du col, du côlon, du poumon, du pancréas, de la prostate, de la tête et du cou, ou de l'estomac, de préférence dans lequel la tumeur est un cancer induit par un virus.

13. Vecteur d'expression d'ADN, mélange de vecteurs d'expression d'ADN, ou kit pour une utilisation selon la revendication 11 ou 12, administré par injection intradermique, de préférence en combinaison avec une électroporation.


### SEQUENCE LISTING

<110> Invectys
<120> Polyepitope constructs for use in immunotherapy
<130> B2109
<160> 37
<170> PatentIn version 3.5
<210> 1
   <211> 15
   <212> PRT
   <213> Homo sapiens
<400> 1
   Pro Ala Ala Phe Arg Ala Leu Val Ala Gln Cys Leu Val Cys Val
   1               5                   10                  15
<210> 2
   <211> 15
   <212> PRT
   <213> Homo sapiens
<400> 2
   Lys Ser Val Trp Ser Lys Leu Gln Ser Ile Gly Ile Arg Gln His
   1               5                   10                  15
<210> 3
   <211> 15
   <212> PRT
   <213> Homo sapiens
<400> 3
   Gly Thr Ala Phe Val Gln Met Pro Ala His Gly Leu Phe Pro Trp
   1               5                   10                  15
<210> 4
   <211> 15
   <212> PRT
   <213> Homo sapiens
<400> 4
   Ser Leu Cys Tyr Ser Ile Leu Lys Ala Lys Asn Ala Gly Met Ser
   1               5                   10                  15
<210> 5
   <211> 15
   <212> PRT
   <213> Homo sapiens
<400> 5
   Ser Phe Leu Leu Ser Ser Leu Arg Pro Ser Leu Thr Gly Ala Arg
   1               5                   10                  15
<210> 6
   <211> 15
   <212> PRT
   <213> Homo sapiens
<400> 6
   Ser Arg Pro Trp Met Pro Gly Thr Pro Arg Arg Leu Pro Arg Leu
   1               5                   10                  15
<210> 7
   <211> 15
   <212> PRT
   <213> Homo sapiens
<400> 7
   Arg Pro Leu Phe Leu Glu Leu Leu Gly Asn His Ala Gln Cys Pro
   1 5 10 15
<210> 8
   <211> 15
   <212> PRT
   <213> Homo sapiens
<400> 8
   Ala Lys Phe Leu His Trp Leu Met Ser Val Tyr Val Val Glu Leu
   1 5 10 15
<210> 9
   <211> 15
   <212> PRT
   <213> Homo sapiens
<400> 9
   Val Lys Ala Leu Phe Ser Val Leu Asn Tyr Glu Arg Ala Arg Arg
   1 5 10 15
<210> 10
   <211> 15
   <212> PRT
   <213> Homo sapiens
<400> 10
   Glu Leu Tyr Phe Val Lys Val Asp Val Thr Gly Ala Tyr Asp Thr
   1 5 10 15
<210> 11
   <211> 15
   <212> PRT
   <213> Homo sapiens
<400> 11
   Leu Gln Pro Tyr Met Arg Gln Phe Val Ala His Leu Gln Glu Thr
   1 5 10 15
<210> 12
   <211> 15
   <212> PRT
   <213> Homo sapiens
<400> 12
   Phe Leu Arg Phe Met Cys His His Ala Val Arg Ile Arg Gly Lys
   1 5 10 15
<210> 13
   <211> 15
   <212> PRT
   <213> Homo sapiens
<400> 13
   Ala Phe Val Gln Met Pro Ala His Gly Leu Phe Pro Trp Cys Gly
   1               5                   10                  15
<210> 14
   <211> 15
   <212> PRT
   <213> Homo sapiens
<400> 14
   Tyr Ser Ser Tyr Ala Arg Thr Ser Ile Arg Ala Ser Leu Thr Phe
   1               5                   10                  15
<210> 15
   <211> 15
   <212> PRT
   <213> Homo sapiens
<400> 15
   Asn Arg Gly Phe Lys Ala Gly Arg Asn Met Arg Arg Lys Leu Phe
   1               5                   10                  15
<210> 16
   <211> 15
   <212> PRT
   <213> Homo sapiens
<400> 16
   Asn Ile Tyr Lys Ile Leu Leu Leu Gln Ala Tyr Arg Phe His Ala
   1               5                   10                  15
<210> 17
   <211> 15
   <212> PRT
   <213> Homo sapiens
<400> 17
   Pro Thr Phe Phe Leu Arg Val Ile Ser Asp Thr Ala Ser Leu Cys
   1               5                   10                  15
<210> 18
   <211> 15
   <212> PRT
   <213> Homo sapiens
<400> 18
   Arg Val Thr Tyr Val Pro Leu Leu Gly Ser Leu Arg Thr Ala Gln
   1               5                   10                  15
<210> 19
   <211> 15
   <212> PRT
   <213> Homo sapiens
<400> 19
   Leu Gly Ser Leu Arg Thr Ala Gln Thr Gln Leu Ser Arg Lys Leu
   1               5                   10                  15
<210> 20
   <211> 15
   <212> PRT
   <213> Homo sapiens
<400> 20
   Gly Thr Thr Leu Thr Ala Leu Glu Ala Ala Ala Asn Pro Ala Leu
   1               5                   10                  15
<210> 21
   <211> 369
   <212> DNA
   <213> Artificial Sequence
<220>
   <223> pUCPbasic insert sequence
<220>
   <221> CDS
   <222> (13)..(357)
<400> 21
   aagcttgccg cc atg gtg cag aga ggc gat ccc gcc gct ttc cgc gca ctg       51
                 Met Val Gln Arg Gly Asp Pro Ala Ala Phe Arg Ala Leu
                 1               5                   10
   gtc gct cag tgc ctg gtg tgc gtg ccc tgg gac gca aga ctc ttc ttc         99
   Val Ala Gln Cys Leu Val Cys Val Pro Trp Asp Ala Arg Leu Phe Phe
       15                  20                  25
   tac cgg aag tcc gtg tgg tcc aag ctg cag agt atc gga att agg cag          147
   Tyr Arg Lys Ser Val Trp Ser Lys Leu Gln Ser Ile Gly Ile Arg Gln
   30                  35                  40                  45
   cac ctg aaa agg gtt cag gac gag gcc ctg gga ggt acc gcc ttc gtc          195
   His Leu Lys Arg Val Gln Asp Glu Ala Leu Gly Gly Thr Ala Phe Val
                   50                  55                  60
   cag atg cct gcc cac ggc ctg ttt cct tgg tgc ggt ctg ctc ctg atc          243
   Gln Met Pro Ala His Gly Leu Phe Pro Trp Cys Gly Leu Leu Leu Ile
               65                  70                  75
   agc gat act gct agc ctg tgt tac tcc atc ctg aaa gct aag aat gcc          291
   Ser Asp Thr Ala Ser Leu Cys Tyr Ser Ile Leu Lys Ala Lys Asn Ala
           80                  85                  90
   gga atg tca ctt ggc gca aag gga ggt aaa cca att cct aac ccc ctc          339
   Gly Met Ser Leu Gly Ala Lys Gly Gly Lys Pro Ile Pro Asn Pro Leu
       95                  100                 105
   ttg ggc ttg gac tca aca tgataactcg ag                                    369
   Leu Gly Leu Asp Ser Thr
   110                 115
<210> 22
   <211> 115
   <212> PRT
   <213> Artificial Sequence
<220>
   <223> Synthetic Construct
<400> 22
   Met Val Gln Arg Gly Asp Pro Ala Ala Phe Arg Ala Leu Val Ala Gln
   1               5                   10                  15
   Cys Leu Val Cys Val Pro Trp Asp Ala Arg Leu Phe Phe Tyr Arg Lys
               20                  25                  30
   Ser Val Trp Ser Lys Leu Gln Ser Ile Gly Ile Arg Gln His Leu Lys
           35                  40                  45
   Arg Val Gln Asp Glu Ala Leu Gly Gly Thr Ala Phe Val Gln Met Pro
       50                  55                  60
   Ala His Gly Leu Phe Pro Trp Cys Gly Leu Leu Leu Ile Ser Asp Thr
   65                  70                  75                  80
   Ala Ser Leu Cys Tyr Ser Ile Leu Lys Ala Lys Asn Ala Gly Met Ser
                   85                  90                  95
   Leu Gly Ala Lys Gly Gly Lys Pro Ile Pro Asn Pro Leu Leu Gly Leu
               100                 105                 110
   Asp Ser Thr
           115
<210> 23
   <211> 369
   <212> DNA
   <213> Artificial Sequence
<220>
   <223> pUCP2(4x) insert sequence
<220>
   <221> CDS
   <222> (13)..(369)
<400> 23
   aagcttgccg cc atg gtg cag agg gga gac aag tct gtg tgg tcc aag ctg       51
                 Met Val Gln Arg Gly Asp Lys Ser Val Trp Ser Lys Leu
                 1               5                   10
   cag tcc atc gga atc agg cag cat ccc tgg gac gca cgg ttg ttc ttt         99
   Gln Ser Ile Gly Ile Arg Gln His Pro Trp Asp Ala Arg Leu Phe Phe
       15                  20                  25
   tac cgg aag tcc gtc tgg agc aag ctc cag tcc att ggg atc cgc cag        147
   Tyr Arg Lys Ser Val Trp Ser Lys Leu Gln Ser Ile Gly Ile Arg Gln
   30                  35                  40                  45
   cac ctg aag cgg gtg cag gat gag gct ctc ggc aag agt gtc tgg tca        195
   His Leu Lys Arg Val Gln Asp Glu Ala Leu Gly Lys Ser Val Trp Ser
                   50                  55                  60
   aag ctg caa agc atc ggc att agg cag cac tgc ggg ctc ctt ttg atc        243
   Lys Leu Gln Ser Ile Gly Ile Arg Gln His Cys Gly Leu Leu Leu Ile
               65                  70                  75
   agc gac acc gcc aag tcc gtg tgg agc aaa ctg cag agc atc ggg att        291
   Ser Asp Thr Ala Lys Ser Val Trp Ser Lys Leu Gln Ser Ile Gly Ile
           80                  85                  90
   aga cag cac ctc ggc gcc aag ggg ggg aaa cct att cca aac ccc ctg        339
   Arg Gln His Leu Gly Ala Lys Gly Gly Lys Pro Ile Pro Asn Pro Leu
       95                  100                 105
   ctg ggc ctg gat tct aca tga taa ctc gag                                369
   Leu Gly Leu Asp Ser Thr         Leu Glu
   110                 115
<210> 24
   <211> 115
   <212> PRT
   <213> Artificial Sequence
<220>
   <223> Synthetic Construct
<400> 24
   Met Val Gln Arg Gly Asp Lys Ser Val Trp Ser Lys Leu Gln Ser Ile
   1               5                   10                  15
   Gly Ile Arg Gln His Pro Trp Asp Ala Arg Leu Phe Phe Tyr Arg Lys
               20                  25                  30
   Ser Val Trp Ser Lys Leu Gln Ser Ile Gly Ile Arg Gln His Leu Lys
           35                  40                  45
   Arg Val Gln Asp Glu Ala Leu Gly Lys Ser Val Trp Ser Lys Leu Gln
       50                  55                  60
   Ser Ile Gly Ile Arg Gln His Cys Gly Leu Leu Leu Ile Ser Asp Thr
   65                  70                  75                  80
   Ala Lys Ser Val Trp Ser Lys Leu Gln Ser Ile Gly Ile Arg Gln His
                   85                  90                  95
   Leu Gly Ala Lys Gly Gly Lys Pro Ile Pro Asn Pro Leu Leu Gly Leu
               100                 105                 110
   Asp Ser Thr
           115
<210> 25
   <211> 360
   <212> DNA
   <213> Artificial Sequence
<220>
   <223> pDE7 insert sequence
<220>
   <221> CDS
   <222> (13)..(348)
<400> 25
   aagcttgccg cc atg cca gga gat aca cct aca ttg cat gaa tat atg tta        51
                 Met Pro Gly Asp Thr Pro Thr Leu His Glu Tyr Met Leu
                 1               5                   10
   gat ttg caa cca gag aca act gat ctc tac ggt tat gag caa tta aat          99
   Asp Leu Gln Pro Glu Thr Thr Asp Leu Tyr Gly Tyr Glu Gln Leu Asn
       15                  20                  25
   gac agc tca gag gag gag gat gaa ata gat ggt cca gct gga caa gca         147
   Asp Ser Ser Glu Glu Glu Asp Glu Ile Asp Gly Pro Ala Gly Gln Ala
   30                  35                  40                  45
   gca ccg gac aga gcc cat tac aat att gta acc ttt tgt tgc aag tgt         195
   Ala Pro Asp Arg Ala His Tyr Asn Ile Val Thr Phe Cys Cys Lys Cys
                   50                  55                  60
   gac tct acg ctt cgg aga tgc gta caa agc aca cac gta gac att cgt         243
   Asp Ser Thr Leu Arg Arg Cys Val Gln Ser Thr His Val Asp Ile Arg
               65                  70                  75
   act ttg gaa gac ctg tta atg ggc aca cta gga att gtg tgc ccc atc         291
   Thr Leu Glu Asp Leu Leu Met Gly Thr Leu Gly Ile Val Cys Pro Ile
           80                  85                  90
   tgt tct cag aaa cca ggt aaa cca att cct aac ccc ctc ttg ggc ttg         339
   Cys Ser Gln Lys Pro Gly Lys Pro Ile Pro Asn Pro Leu Leu Gly Leu
       95                  100                 105
   gac tca aca tgataactcg ag                                               360
   Asp Ser Thr
   110
<210> 26
   <211> 112
   <212> PRT
   <213> Artificial Sequence
<220>
   <223> Synthetic Construct
<400> 26
   Met Pro Gly Asp Thr Pro Thr Leu His Glu Tyr Met Leu Asp Leu Gln
   1               5                   10                  15
   Pro Glu Thr Thr Asp Leu Tyr Gly Tyr Glu Gln Leu Asn Asp Ser Ser
               20                  25                  30
   Glu Glu Glu Asp Glu Ile Asp Gly Pro Ala Gly Gln Ala Ala Pro Asp
           35                  40                  45
   Arg Ala His Tyr Asn Ile Val Thr Phe Cys Cys Lys Cys Asp Ser Thr
       50                  55                  60
   Leu Arg Arg Cys Val Gln Ser Thr His Val Asp Ile Arg Thr Leu Glu
   65                  70                  75                  80
   Asp Leu Leu Met Gly Thr Leu Gly Ile Val Cys Pro Ile Cys Ser Gln
                   85                  90                  95
   Lys Pro Gly Lys Pro Ile Pro Asn Pro Leu Leu Gly Leu Asp Ser Thr
               100                 105                 110
<210> 27
   <211> 15
   <212> PRT
   <213> Papillomavirus sp
<400> 27
   His Glu Tyr Met Leu Asp Leu Gln Pro Glu Thr Thr Asp Leu Tyr
   1               5                   10                  15
<210> 28
   <211> 15
   <212> PRT
   <213> Papillomavirus sylvilagi
<400> 28
   His Val Asp Ile Arg Thr Leu Glu Asp Leu Leu Met Gly Thr Leu
   1               5                   10                  15
<210> 29
   <211> 9
   <212> PRT
   <213> Papillomavirus sylvilagi
<400> 29
   Thr Leu His Glu Tyr Met Leu Asp Leu
   1               5
<210> 30
   <211> 10
   <212> PRT
   <213> Papillomavirus sylvilagi
<400> 30
   Tyr Met Leu Asp Leu Gln Pro Glu Thr Thr
   1 5 10
<210> 31
   <211> 9
   <212> PRT
   <213> Papillomavirus sylvilagi
<400> 31
   Asp Leu Gln Pro Glu Thr Thr Asp Leu
   1 5
<210> 32
   <211> 10
   <212> PRT
   <213> Papillomavirus sylvilagi
<400> 32
   Thr Leu Glu Asp Leu Leu Met Gly Thr Leu
   1 5 10
<210> 33
   <211> 10
   <212> PRT
   <213> Papillomavirus sylvilagi
<400> 33
   Asp Leu Leu Met Gly Thr Leu Gly Ile Val
   1 5 10
<210> 34
   <211> 9
   <212> PRT
   <213> Papillomavirus sylvilagi
<400> 34
   Leu Leu Met Gly Thr Leu Gly Ile Val
   1 5
<210> 35
   <211> 8
   <212> PRT
   <213> Papillomavirus sylvilagi
<400> 35
   Thr Leu Gly Ile Val Cys Pro Ile
   1               5
<210> 36
   <211> 20
   <212> DNA
   <213> Artificial Sequence
<220>
   <223> primer
<400> 36
   taatacgact cactataggg 20
<210> 37
   <211> 18
   <212> DNA
   <213> Artificial Sequence
<220>
   <223> primer
<400> 37
   tagaaggcac agtcgagg 18